(19) **Europäisches Patentamt**
**European Patent Office**
**Office européen des brevets**

(11) **EP 2 618 145 B1**

(12) **EUROPEAN PATENT SPECIFICATION**

(45) Date of publication and mention
of the grant of the patent:
**23.11.2016 Bulletin 2016/47**

(51) Int Cl.:
*G01N 33/497* *(2006.01)*    *A61B 10/00* *(2006.01)*
*A61K 49/00* *(2006.01)*    *G01N 33/00* *(2006.01)*

(21) Application number: **11818231.0**

(86) International application number:
**PCT/JP2011/068714**

(22) Date of filing: **18.08.2011**

(87) International publication number:
**WO 2012/023590 (23.02.2012 Gazette 2012/08)**

(54) **METHOD FOR QUANTITATIVE MEASUREMENT OF GASTRIC ACIDITY USING 13C CARBONATE SALT**

VERFAHREN ZUR QUANTITATIVEN MAGENSÄUREMESSUNG MITHILFE VON 13C-CARBONAT-SALZ

PROCÉDÉ DE MESURE QUANTITATIVE DE L'ACIDITÉ GASTRIQUE À L'AIDE D'UN SEL CARBONATE MARQUÉ AU C13

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.08.2010 JP 2010184486**

(43) Date of publication of application:
**24.07.2013 Bulletin 2013/30**

(73) Proprietor: **Otsuka Pharmaceutical Co., Ltd.**
**Tokyo 101-8535 (JP)**

(72) Inventors:
• **INADA, Makoto**
**Osaka-shi**
**Osaka 541-0045 (JP)**
• **KUNIZAKI, Jun-ichi**
**Osaka-shi**
**Osaka 541-0045 (JP)**
• **TOBITA, Kazuki**
**Osaka-shi**
**Osaka 541-0045 (JP)**
• **AKAMATSU, Suguru**
**Osaka-shi**
**Osaka 541-0045 (JP)**
• **IIZUKA, Shinji**
**Osaka-shi**
**Osaka 541-0045 (JP)**

(74) Representative: **Hoffmann Eitle**
**Patent- und Rechtsanwälte PartmbB**
**Arabellastraße 30**
**81925 München (DE)**

(56) References cited:
**EP-A1- 1 285 668**    **WO-A1-01/82979**
**WO-A1-01/97863**    **WO-A2-2007/007100**
**WO-A2-2008/028116**    **CN-A- 101 262 891**
**JP-A- 2008 044 889**    **JP-A- 2008 531 068**
**JP-A- 2008 538 275**    **JP-A- 2009 515 139**
**JP-A- 2010 502 194**    **JP-A- 2011 106 846**

• **MICHAEL R.I. CLOUGH, ANTHONY T.R. AXON:**
**"The Calcium Carbonate Breath Test, a noninvasive test of stimulated gastric acid secretion: preliminary communication", EUR J GASTROENTEROL HEPATOL., vol. 21, no. 3, March 2009 (2009-03), pages 266-272, XP008166715, DOI: 10.1097/MEG.0b013e328321837e**
• **Klaus Wetzel ET AL: "13C-Breath Tests in Medical Research and Clinical Diagnosis", , 1 May 2005 (2005-05-01), XP055096455, Retrieved from the Internet: URL:http://www.fan-gmbh.de/docs/13c-breath tests.pdf [retrieved on 2014-01-15]**

**(Cont. next page)**

- ERIKA SUGIYAMA ET AL.: 'I Naiyo Haisetsu Sokudo Hyoka o Mokuteki to shita Shinki Koki Shiken Probe 13C-uracil ni Kansuru Yakubutsu Dotaigakuteki Kento' JOURNAL OF SMOOTH MUSCLE RESEARCH vol. 14, no. 1, 11 June 2010, page J-7

- V SAVARINO ET AL: "The 13C urea breath test in the diagnosis of Helicobacter pylori infection", GUT, vol. 45, no. Supplement 1, 1 July 1999 (1999-07-01), pages i18-i22, XP055208230, ISSN: 0017-5749, DOI: 10.1136/gut.45.2008.i18

**Description**

Technical Field

The present invention relates to a method for measuring the gastric acidity of a mammal using a $^{13}C$-labeled carbonate compound. More specifically, the present invention relates to a method for non-invasively and quantitatively measuring the gastric acidity of a mammal using expired air excreted after the administration of a $^{13}C$-labeled carbonate compound.

The present invention also relates to a method for diagnosing a disease relating to gastric acid secretion by measuring gastric acidity tendency (hyperacidity, normal, hypoacidity, or anacidity), and a method for measuring the effect of a drug that has the action of suppressing gastric acid (hereinafter referred to as a "gastric acid reducer").

Further, the present invention relates to, from another perspective as examples of the application of the above methods, a method for evaluating the enzyme activity (metabolic capacity) of CYP2C19 alone or both CYP2C19 and CYP3A4 in a subject; and a method for evaluating the effect of a drug metabolized by CYP2C19 alone or both CYP2C19 and CYP3A4 on a subject, and the susceptibility of a subject to the drug.

Background Art

A large number of drugs are synthesized in the form of organic acids or organic bases. It is known that some of these organic acids and organic bases are influenced by gastric acidity, causing large changes in bioavailability; as a result, they do not produce the expected pharmacological effects, or cause unexpected and severe side effects. Further, in today's aging society, the number of patients with hypoacidity or anacidity is said to be rapidly increasing.

In the case of such patients, it is believed that measuring the tendency (hyperacidity, normal, hypoacidity, anacidity, or the like) of gastric acidity (HCl concentration x amount of gastric juice) before medication provides very useful information for selecting a drug, and predicting the therapeutic and side effects of the drug. The advent of gastric acid secretion inhibitors, such as $H_2$-antagonists and proton pump inhibitors (PPIs), has greatly contributed to the treatment of gastric and duodenal ulcers. However, recrudescence or recurrence (in particular, recurrence of reflux esophagitis) after treatment has become a problem in recent years; therefore, medicinal treatment methods for gastric and duodenal ulcers, including revisions of treatment methods, are attracting attention as a subject to be examined in the medical field. Treatment with a gastric acid secretion inhibitor suppresses gastric acid secretion, and its therapeutic effect can be evaluated by measuring basic gastric acid secretion. The recurrence of reflux esophagitis is a rebound phenomenon caused when the administration of a gastric acid secretion inhibitor is discontinued, and is presumed to be predictable to some extent by measuring gastric acid output.

Thus, the measurement of gastric acidity presumably makes it possible to predict the therapeutic and side effects of a drug to some extent. Further, since the gastric acidity tendency has become clear in some diseases (e.g., gastric ulcer, duodenal ulcer, gastric cancer, chronic gastritis, liver/biliary tract/pancreatic disease, pernicious anemia, vitamin B complex deficiency disease, pyloric stenosis, Zollinger-Ellison syndrome, and the like), it is believed that the measurement of gastric acidity will find wide application in diagnosing diseases.

Known methods for measuring gastric acid output include a method comprising sucking acid from the stomach through a naso-gastric tube, while giving a stimulus to promote secretion. However, this method is not practical since it is invasive, and imposes physical and mental strain on a subject. Proposed as a non-invasive method is a method comprising orally administering a large quantity of a water-insoluble carbonate containing an isotope to a subject, and measuring the amount of gastric secretion from the content of the isotope in carbon dioxide excreted in expired air (Patent Literature 1). However, this method measures the content of the isotope in carbon dioxide produced as a result of complete neutralization of gastric acidity; accordingly, a subject must be given an excess quantity of the carbonate relative to gastric acid volume. Moreover, before collecting expired air, it is necessary to wait for at least 60 minutes (preferably at least 150 minutes), which is the time required for the gastric acid to be completely neutralized with the administered carbonate plus the time for the isotope content in carbon dioxide in the expired air to be stabilized; accordingly, the measurement problematically requires time.

Also proposed is a method comprising orally administering an isotope-containing composition to a subject and non-invasively measuring gastric pH from the amount of the isotope in carbon dioxide excreted in expired air, as in the above method (Patent Literature 2). However, this method is based on the finding that when a composition containing an isotope-labeled compound is covered with a pH-dependent soluble base, such as an enteric base or a gastro-soluble base, the behavior of the isotope in carbon dioxide excreted from the body changes according to the gastric pH, and there is a constant relation between the gastric pH and the excretion behavior of the labeled compound. Thus, this method requires the use of an administration preparation covered with a pH-dependent soluble base.

CN 101 262 891 A and WO 2007/007100 A2 relates to a method of measuring the amount of gastric secretion in a mammal involves administering a substance or formulation containing an excess quantity of a water-insoluble carbonate to the mammal to react with acid in the stomach, in which the insoluble carbonate is enriched with at least one isotope selected from $^{13}C$, $^{14}C$, $^{17}O$ and $^{18}OO$ in a known amount. The content of the or each selected isotope in the exhaled carbon dioxide is allowed to stabilise before obtaining a sample of exhaled air containing carbon dioxide, and determining the content of the selected or each selected isotope in the exhaled carbon dioxide.

Eur. J. Gastroenterol. Hepatol. 21(3), 2009, 266-272 relates to a method of measuring gastric acid secretion noninvasively wherein $^{13}C$-labelled calcium carbonate is ingested by a fasting participant. The carbonate neutralizes gastric acid to produce $^{13}CO_2$, which is expired in the breath. Measurement of the excess $^{13}CO_2$ in the breath allows back-calculation of the amount of gastric acid secreted during the test, provided that other variables affecting $CO_2$ production and «excretion are minimized or controlled for.

WO 2008/028116 A2 relates to a method of determining and assessing cytochrome P450 2C19 related (CYP2C19) metabolic capacity in an individual mammalian subject via a breath assay, by determining the relative amount of $^{13}CO_2$ exhaled by the subject upon intravenous or oral administration of a $^{13}C$ labeled CYP2C19 substrate compound.

Gut 1999; 45(Suppl I), I18-I22 discloses an invention that is directed to diagnosis of H. Pylori infection using a $^{13}C$-labeled urea expiration test. Urease activity, which serves as an index for *H. Pylori* infection, is evaluated by measuring the amount of $^{13}CO_2$ in expired air produced through degradation of $^{13}C$-labeled urea by urease, in order to diagnose *H. Pylori* infection.

Citation List

Patent Literature

PTL 1: Japanese Unexamined Patent Publication No. 2009-515139
PTL 2: WO01/97863A1

Summary of Invention

Technical Problem

An object of the present invention is to provide a method for quantitatively measuring the gastric acidity of a mammal, including a human, in a non-invasive and simple manner, using a $^{13}C$-labeled carbonate compound.

Another object of the present invention is to provide a method for diagnosing a disease relating to gastric acid secretion by measuring gastric acidity tendency (hyperacidity, normal, hypoacidity, or anacidity), and a method for measuring the effect of a gastric acid reducer (e.g., gastric acid secretion inhibitors, such as proton pump inhibitors and $H_2$ blockers; and drugs for neutralizing gastric acid, such as antacids). An additional object of the present invention is to provide, as examples of the application of the above methods, a method for evaluating the enzyme activity (metabolic capacity) of CYP2C19 alone or both CYP2C19 and CYP3A4 in a subject; and a method for evaluating the effect of a drug metabolized by CYP2C19 alone or both CYP2C19 and CYP3A4 on a subject, and the susceptibility of a subject to the drug.

Solution to Problem

To solve the above problems, the present inventors analyzed $^{13}CO_2$ excretion behavior in expired air obtained after orally administering a $^{13}C$-labeled carbonate compound to mammals, and found that regardless of the degree of gastric acidity of the mammals, there is a linear correlation passing through the origin between the dose of the $^{13}C$-labeled carbonate compound and $\Delta^{13}C(‰)$ at a point in time for collecting the expired air. Here, "$\Delta^{13}C(‰)$" means the difference $[\Delta^{13}C(‰) = (\delta^{13}C)_t - (\delta^{13}C)_0]$ between the ratio ("$(\delta^{13}C)_t$") of $^{13}CO_2$ amount to $^{12}CO_2$ amount in the expired air at a point in time for collecting the expired air (t), and the ratio ("$(\delta^{13}C)_0$") of $^{13}CO_2$ amount to $^{12}CO_2$ amount in the expired air before the administration of the $^{13}C$-labeled carbonate compound. In addition, "dose" means the amount of the $^{13}C$-labeled carbonate compound administered to a mammalian subject; and encompasses both the dose (number of moles, weight) of the $^{13}C$-labeled carbonate compound administered to 1 body of a mammalian subject, and the dose (number of moles, weight) of the $^{13}C$-labeled carbonate compound administered per kg body weight of a mammalian subject.

The present inventors also found that when the dose exceeds a certain amount, the $\Delta^{13}C(‰)$ curve versus the dose of the $^{13}C$-labeled carbonate compound reaches a nearly constant value ($\Delta^{13}C(‰)$ plateau value) according to the degree of gastric acidity of mammals; that the dose at which the $\Delta^{13}C(‰)$ reaches a plateau (corresponding to the above "certain amount") varies depending on the gastric acidity of the mammals; and that there is a corresponding relation between the dose and the gastric acidity. Based on these findings, the present inventors confirmed that the gastric acidity of each subject can be measured and evaluated using the $\Delta^{13}C(‰)$ plateau value as an index. As described above, regardless of the degree of gastric acidity of mammals, there is a linear correlation passing through the origin between the dose of the $^{13}C$-labeled carbonate compound and the $\Delta^{13}C(‰)$ until the $\Delta^{13}C(‰)$ reaches a plateau.

Further, the present inventors confirmed that, as in the case of the above $\Delta^{13}C(‰)$, when the dose exceeds a certain amount, the "area under the $\Delta^{13}C(‰)$-time curve" (AUC) versus the dose of the $^{13}C$-labeled carbonate compound reaches a nearly constant value (AUC plateau value) according to the degree of gastric acidity of mammals; that the dose at which the AUC reaches a plateau (corresponding to the above "certain amount") varies depending on the gastric acidity of the mammals, and there is a corresponding relation between the dose and the gastric acidity; and that, as in the case of the $\Delta^{13}C(‰)$, the gastric acidity of each subject can be measured and evaluated using the AUC plateau value as an index. Furthermore, as in the case of the $\Delta^{13}C(‰)$, regardless of the degree of gastric acidity of mammals, there is a linear correlation passing through the origin between the dose of the $^{13}C$-labeled carbonate compound and the AUC until the AUC reaches a plateau.

As described above, the method of the present invention makes it possible to quantitatively measure and evaluate the gastric acidity of a subject non-invasively in a short period of time, without restraining the subject for a long period of time, by using the $\Delta^{13}C(‰)$ as an index.

The present invention was accomplished based on these findings, and includes the following embodiments.

(I) Method for measuring gastric acidity of a mammal

(I-1) A method for measuring gastric acidity of a mammal comprising the steps of:

(1) using, as a test sample, expired air of a mammalian subject excreted at any point in time within 30 minutes after oral administration of a predetermined amount of a $^{13}C$-labeled carbonate compound, measuring behavior of $^{13}CO_2$ excreted in the expired air;

(2) comparing the behavior of $^{13}CO_2$ (measured $^{13}CO_2$ behavior) obtained in step (1) with the behavior of corresponding $^{13}CO_2$ (reference $^{13}CO_2$ behavior) that has been obtained beforehand in a control mammal; and

(3) determining gastric acidity of the mammalian subject based on a difference between the reference $^{13}CO_2$ behavior and the measured $^{13}CO_2$ behavior obtained above.

(I-2) The method according to Item (I-1), wherein the behavior of $^{13}CO_2$ is $\Delta^{13}C(‰)_t$ (t is an expired air collection time, within 30 minutes) obtained from expired air of a mammalian subject collected at any point in time within 30 minutes after oral administration of a $^{13}C$-labeled carbonate compound.

(I-3) The method according to Item (I-1) or (I-2), wherein the predetermined amount is 10 mg to 5 g.

(I-4) The method according to any one of Items (I-1) to (I-3), wherein the $^{13}C$-labeled carbonate compound is at least one carbonate compound selected from the group consisting of alkali metal carbonates, alkaline earth metal carbonates, ammonium carbonate, alkali metal hydrogencarbonates, and ammonium hydrogencarbonate.

(I-5) The method according to any one of Items (I-1) to (I-3), wherein the $^{13}C$-labeled carbonate compound is at least one carbonate compound selected from the group consisting of sodium carbonate, potassium carbonate, calcium carbonate, magnesium carbonate, barium carbonate, ammonium carbonate, potassium hydrogencarbonate, sodium hydrogencarbonate, and ammonium hydrogencarbonate.

(I-6) The method according to any one of Items (I-1) to (I-5), wherein the expired air used as a test sample is expired air of a mammalian subject excreted at any point in time within 20 minutes, preferably 15 minutes after oral administration of a $^{13}C$-labeled carbonate compound.

(I-7) The method according to any one of Items (I-2) to (I-6), wherein the control mammal used in comparing step (2) is a mammal having normal gastric acidity, and determination step (3) is a step of determining that the gastric acidity of the mammalian subject is the same as or higher than the gastric acidity of the control mammal when the measured $^{13}CO_2$ behavior and the reference $^{13}CO_2$ behavior are the same, or that the gastric acidity of the mammalian subject is lower than the gastric acidity of the control mammal when the measured $^{13}CO_2$ behavior is lower than the reference $^{13}CO_2$ behavior.

(II) Method for measuring an effect of a gastric acid reducer

(II-1) A method for measuring an effect of a gastric acid reducer on a mammal, the method comprising the following steps (1) to (4):

(1) using, as a test sample, expired air of a mammalian subject excreted at any point in time within 30 minutes after oral administration of a predetermined amount of a $^{13}C$-labeled carbonate compound, the oral administration being performed after administration of a gastric acid reducer, measuring behavior of $^{13}CO_2$ excreted in the expired air,

(2) comparing the behavior of $^{13}CO_2$ (measured $^{13}CO_2$ behavior) obtained in step (1) with the behavior of corresponding $^{13}CO_2$ (reference $^{13}CO_2$ behavior) measured in a mammal (control mammal) to which a predetermined amount of a $^{13}C$-labeled carbonate compound has been orally administered beforehand without administering the gastric acid reducer;

(3) determining gastric acidity of the mammalian subject based on a difference between the reference $^{13}CO_2$ behavior and the measured $^{13}CO_2$ behavior obtained above; and

(4) determining the effect of the gastric acid reducer on the mammalian subject using the gastric acidity of the mammalian subject obtained above as an index.

(II-2) The method according to Item (II-1), wherein the behavior of $^{13}CO_2$ is $\Delta^{13}C(‰)_t$ (t is an expired air collection time, within 30 minutes) obtained from expired air of a mammalian subject collected at any point in time within 30 minutes after oral administration of a $^{13}C$-labeled carbonate compound.

(II-3) The method according to Item (II-1) or (II-2), wherein the predetermined amount is 10 mg to 5 g.

(II-4) The method according to any one of Items (II-1) to (II-3), wherein the $^{13}C$-labeled carbonate compound is at least one carbonate compound selected from the group consisting of alkali metal carbonates, alkaline earth metal carbonates, ammonium carbonate, alkali metal hydrogencarbonates, and ammonium hydrogencarbonate.

(II-5) The method according to any one of Items (II-1) to (II-3), wherein the $^{13}C$-labeled carbonate compound is at least one carbonate compound selected from the group consisting of sodium carbonate, potassium carbonate, calcium carbonate, magnesium carbonate, barium carbonate, ammonium carbonate, potassium hydrogencarbonate, sodium hydrogencarbonate, and ammonium hydrogencarbonate.

(II-6) The method according to any one of Items (II-1) to (II-5), wherein the expired air used as a test sample is expired air of a mammalian subject excreted at any point in time within 20 minutes, preferably 15 minutes after oral administration of a $^{13}C$-labeled carbonate compound.

(II-7) The method according to any one of Items (II-1) to (II-6), wherein step (3) is a step of determining that the gastric acidity of the mammalian subject is the same as or higher than the gastric acidity of the control mammal when the measured $^{13}CO_2$ behavior and the reference $^{13}CO_2$ behavior are the same, or that the gastric acidity of the mammalian subject is lower than the gastric acidity of the control mammal when the measured $^{13}CO_2$ behavior is lower than the reference $^{13}CO_2$ behavior.

(II-8) The method according to Item (II-7), wherein step (4) is a step of determining that the administered gastric acid reducer has no effect on the mammalian subject when the gastric acidity of the mammalian subject measured in step (3) is the same as or higher than the gastric acidity of the control mammal; or that the administered gastric acid reducer has an effect on the mammalian subject when the gastric acidity of the mammalian subject measured in step (3) is lower than the gastric acidity of the control mammal.

(II-9) The method according to any one of Items (II-1) to (II-8), wherein the gastric acid reducer is a proton pump inhibitor, an $H_2$ blocker, or an antacid.

(II-10) The method according to Item (II-9) wherein the proton pump inhibitor is at least one member selected from the group consisting of omeprazole, lansoprazole, pantoprazole, rabeprazole, and esomeprazole; the $H_2$ blocker is at least one member selected from the group consisting of ranitidine, cimetidine, famotidine, nizatidine, lafutidine, and roxatidine acetate hydrochloride; and the antacid is at least one member selected from the group consisting of magnesium hydroxide, anhydrous dibasic calcium phosphate, precipitated calcium carbonate, sodium hydrogencarbonate, and magnesium oxide.

(III) Method for evaluating enzyme activity (metabolic capacity) of CYP2C19 alone or both CYP2C19 and CYP3A4 in a mammalian subject, effect of a drug metabolized by CYP2C19 alone or both CYP2C19 and CYP3A4 on a mammalian subject, or/and susceptibility of a mammalian subject to the drug

(III-1) A method for evaluating enzyme activity (metabolic capacity) of CYP2C19 alone or both CYP2C19 and CYP3A4 in a mammalian subject, effect of a drug metabolized by CYP2C19 alone or both CYP2C19 and CYP3A4 on the mammalian subject, or/and susceptibility of the mammalian subject to the drug, the method comprising the following steps (1) to (4):

(1) using, as a test sample, expired air of a mammalian subject excreted at any point in time within 30 minutes after oral administration of a predetermined amount of a $^{13}C$-labeled carbonate compound, the oral administration being performed after administration of omeprazole or lansoprazole, measuring behavior of $^{13}CO_2$ excreted in the expired air,

(2) comparing the behavior of $^{13}CO_2$ (measured $^{13}CO_2$ behavior) obtained in step (1) with behavior of corresponding $^{13}CO_2$ (reference $^{13}CO_2$ behavior) measured in a mammal (control mammal) to which a predetermined amount of a $^{13}C$-labeled carbonate compound has been orally administered beforehand without administering omeprazole and lansoprazole;

(3) determining the gastric acidity of the mammalian subject based on a difference between the reference $^{13}CO_2$ behavior and the measured $^{13}CO_2$ behavior obtained above; and

(4) determining the enzyme activity (metabolic capacity) of CYP2C19 alone or both CYP2C19 and CYP3A4 in the mammalian subject, effect of a drug metabolized by CYP2C19 alone or both CYP2C19 and CYP3A4 on the mammalian subject, or/and susceptibility of the mammalian subject to the drug, using the gastric acidity of the mammalian subject obtained above as an index.

(III-2) The method according to Item (III-1), wherein the behavior of $^{13}CO_2$ is $\Delta^{13}C(‰)_t$ (t is an expired air collection time, within 30 minutes) obtained from expired air of a mammalian subject collected at any point in time within 30 minutes after oral administration of a $^{13}C$-labeled carbonate compound.

(III-3) The method according to Item (III-1) or (III-2), wherein the predetermined amount is 10 mg to 5 g.

(III-4) The method according to any one of Items (III-1) to (III-3), wherein the $^{13}C$-labeled carbonate compound is at least one carbonate compound selected from the group consisting of alkali metal carbonates, alkaline earth metal carbonates, ammonium carbonate, alkali metal hydrogencarbonates, and ammonium hydrogencarbonate.

(III-5) The method according to any one of Items (III-1) to (III-3), wherein the $^{13}C$-labeled carbonate compound is at least one carbonate compound selected from the group consisting of sodium carbonate, potassium carbonate, calcium carbonate, magnesium carbonate, barium carbonate, ammonium carbonate, potassium hydrogencarbonate, sodium hydrogencarbonate, and ammonium hydrogencarbonate.

(III-6) The method according to any one of Items (III-1) to (III-5), wherein the expired air used as a test sample is expired air of a mammalian subject excreted at any point in time within 20 minutes, preferably 15 minutes after oral administration of a $^{13}C$-labeled carbonate compound.

(III-7) The method according to any one of Items (III-1) to (III-6), wherein step (3) is a step of determining that the gastric acidity of the mammalian subject is the same as or higher than the gastric acidity of the control mammal when the measured $^{13}CO_2$ behavior and the reference $^{13}CO_2$ behavior are the same, or that the gastric acidity of the mammalian subject is lower than the gastric acidity of the control mammal when the measured $^{13}CO_2$ is lower than the reference $^{13}CO_2$.

(III-8) The method according to Item (III-7) comprising as step (4) a step of determining enzyme activity (metabolic capacity) of CYP2C19 alone or both CYP2C19 and CYP3A4 in a mammalian subject, wherein step (4) is a step of determining that the enzyme activity (metabolic capacity) of CYP2C19 alone or both CYP2C19 and CYP3A4 in the mammalian subject is normal or high when step (3) determines that the gastric acidity of the mammalian subject is the same as or higher than the gastric acidity of the control mammal; or that the enzyme activity (metabolic capacity) of CYP2C19 alone or both CYP2C19 and CYP3A4 in the mammalian subject is low when step (3) determines that the gastric acidity of the mammalian subject is lower than the gastric acidity of the control mammal.

(III-9) The method according to Item (III-7) or (III-8) comprising as step (4) a step of determining effect of a drug metabolized by CYP2C19 alone or both CYP2C19 and CYP3A4 on a mammalian subject, wherein

(a) in the case where the drug metabolized by CYP2C19 alone or both CYP2C19 and CYP3A4 shows an effect before being metabolized, step (4) is a step of determining that the effect of the drug on the mammalian subject is low when step (3) determines that the gastric acidity of the mammalian subject is the same as or higher than the gastric acidity of the control mammal, or that the effect of the drug on the mammalian subject is high when step (3) determines that the gastric acidity of the mammalian subject is lower than the gastric acidity of the control mammal; or

(b) in the case where the drug metabolized by CYP2C19 alone or both CYP2C19 and CYP3A4 shows an effect by metabolization, step (4) is a step of determining that the effect of the drug on the mammalian subject is high when step (3) determines that the gastric acidity of the mammalian subject is the same as or higher than the gastric acidity of the control mammal, or that the effect of the drug on the mammalian subject is low when step (3) determines that the gastric acidity of the mammalian subject is lower than the gastric acidity of the control mammal.

(III-10) The method according to any one of Items (III-7) to (III-9) comprising as step (4) a step of determining susceptibility of a mammalian subject to a drug metabolized by CYP2C19 alone or both CYP2C19 and CYP3A4, wherein

(a) in the case where the drug metabolized by CYP2C19 alone or both CYP2C19 and CYP3A4 shows an effect before being metabolized, step (4) is a step of determining that the susceptibility of the mammalian subject to the drug is high when step (3) determines that the gastric acidity of the mammalian subject is the same as or higher than the gastric acidity of the control mammal, or that the susceptibility of the mammalian subject to the drug is low when step (3) determines that the gastric acidity of the mammalian subject is lower than the gastric acidity of the control mammal; or

(b) in the case where the drug metabolized by CYP2C19 alone or both CYP2C19 and CYP3A4 shows an effect by metabolization, step (4) is a step of determining that the susceptibility of the mammalian subject to the drug is low when step (3) determines that the gastric acidity of the mammalian subject is the same as or higher than the gastric acidity of the control mammal, or that the susceptibility of the mammalian subject to the drug is high when step (3) determines that the gastric acidity of the mammalian subject is lower than the gastric acidity of the control mammal.

(III-11) The method according to Item (III-9) or (III-10), wherein the drug metabolized by CYP2C19 alone or both CYP2C19 and CYP3A4 is any one of those selected from the group consisting of diazepam, omeprazole, lansoprazole, propranolol, and clopidogrel.

(III-12) The method according to Item (III-9) or (III-10), wherein the drug that exhibits an effect before being metabolized by CYP2C19 alone or both CYP2C19 and CYP3A4 is any one of those selected from the group consisting of diazepam, omeprazole, lansoprazole, and propranolol.

(III-13) The method according to (III-9) or (III-10), wherein the drug that exhibits an effect by being metabolized by CYP2C19 alone or both CYP2C19 and CYP3A4 is clopidogrel.

Advantageous Effects of Invention

The method of the present invention for measuring gastric acidity makes it possible to quantitatively measure gastric acidity in a simple manner by using an expiration test using a $^{13}$C-labeled carbonate compound as an oral preparation, without placing any mental or physical burden on a subject that is a mammal, including a human.

In addition, the method of the present invention makes it possible to diagnose a disease relating to gastric acid secretion for a mammalian subject; to measure and evaluate the effect of a drug relating to gastric acid secretion (e.g., gastric acid secretion inhibitors such as proton pump inhibitors and $H_2$ blockers) or a drug that has the action of neutralizing gastric acid (e.g., antacids) (these drugs are referred to as "gastric acid reducers"); or to easily measure and evaluate the effect of a drug metabolized by CYP2C19 alone or both CYP2C19 and CYP3A4, and the susceptibility to the drug (for example, including deficiencies, etc., in these metabolism enzymes).

For example, in the case where proton pump inhibitors, such as lansoprazole, among drugs relating to gastric acid secretion, are used, the drugs are metabolized by hepatic metabolism enzymes CYP2C19 and CYP3A4. Thus, by measuring the gastric acidity of mammalian subjects after administration of these drugs, the enzyme activity (metabolic capacity) of CYP2C19 and CYP3A4 in the mammalian subjects (reduction or elevation in CYP2C19 and CYP3A4 due to genetic defects or presence of polymorphisms of the hepatic metabolism enzymes) can be measured and evaluated.

Further, from this perspective, the present invention can provide a method for measuring and evaluating the enzyme activity (metabolic capacity) of CYP2C19 alone or both CYP2C19 and CYP3A4 in a mammal, including a human, used as a test subject; and can also provide a method for measuring and evaluating the effect on a mammalian subject of a drug that exhibits its effect before being metabolized by the enzyme or enzymes, or of a drug that exhibits its effect by being metabolized by the enzyme or enzymes (this can also be regarded as the susceptibility of a mammalian subject to these drugs).

Brief Description of Drawings

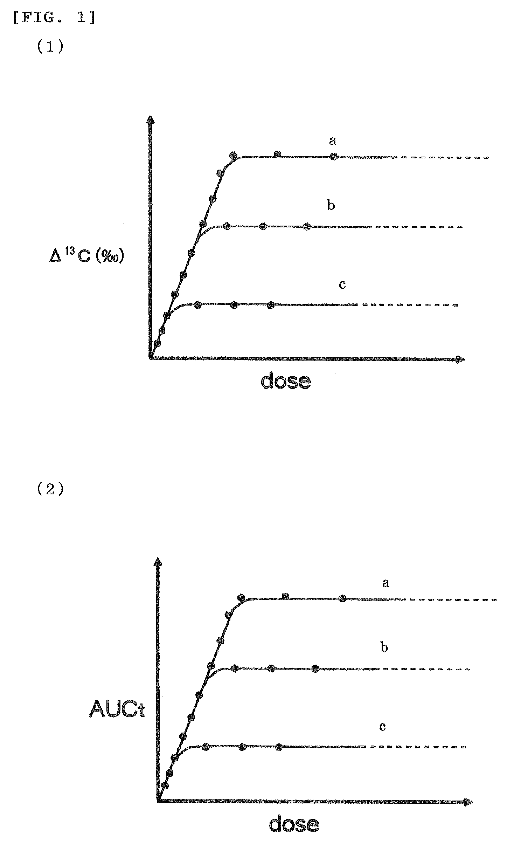

Fig. 1 (1) shows the correlation between each dose of the administration preparation and $\Delta^{13}$C(‰). Fig. 1 (2) shows the correlation between each dose of the administration preparation and the "area under the $\Delta^{13}$C(‰) -time curve" (AUCt). Here, "time" means a time (t) from when the administration preparation is administered to when expired air is collected. In the dose up to a certain amount, linear correlations passing through the origin are shown between the dose and the $\Delta^{13}$C(‰), and between the dose and the AUCt, regardless of the degree of the gastric acidity of the subjects (mammals). It is also shown that the $\Delta^{13}$C(‰) plateau value and the dose thereof at which the $\Delta^{13}$C(‰)

becomes constant and the AUCt plateau value and the dose thereof at which the AUCt becomes constant vary depending on the degree of the gastric acidity of the subjects (mammals) (a: high gastric acidity, b: normal gastric acidity, and c: low gastric acidity).

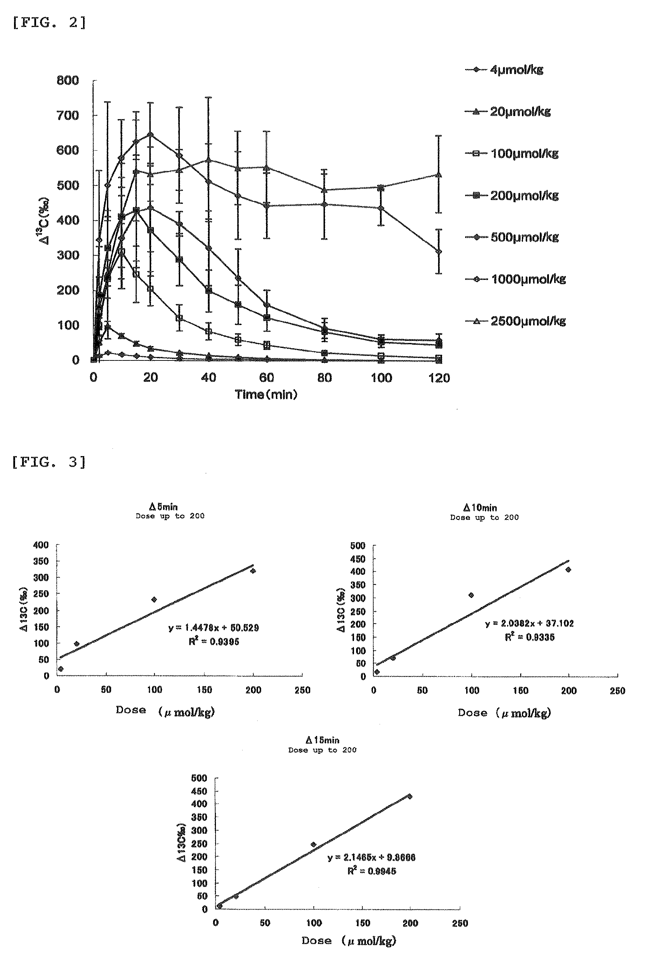

Fig. 2 is a graph showing changes over time in $\Delta^{13}C$(‰) measured from $^{13}CO_2$ in expired air after each of the $^{13}C$-$CaCO_3$ suspensions at various concentrations (2500, 1000, 500, 200, 100, 20, and 4 $\mu$mol/4 mL) was administered to rats in Experimental Example 1. In Fig. 2, the $\Delta^{13}C$(‰) $[(\delta^{13}C_t) - (\delta^{13}C_0)]$ in the expired air is plotted on the ordinate, whereas the expired air collection time (minutes) after the $^{13}C$-$CaCO_3$ administration is plotted on the abscissa. The $(\delta^{13}C_t)$ is a $^{13}CO_2/^{12}CO_2$ concentration ratio in the expired air at each collection point in time (t) after the $^{13}C$-$CaCO_3$ administration. The $(\delta^{13}C_0)$ is a $^{13}CO_2/^{12}CO_2$ concentration ratio in the expired air before the $^{13}C$-$CaCO_3$ administration (0).

Fig. 3 shows correlations between the $^{13}C$-$CaCO_3$ dose ($\mu$mol/kg) of 4 to 200 $\mu$mol/kg and the $\Delta^{13}C$(‰) in the expired air in subjects (mammals: rats) (Experimental Example 1). Fig. 3(1) is a graph showing a correlation between the dose ($\mu$mol/kg) and the $\Delta^{13}C$(‰) in the expired air in the case where the expired air collected 5 minutes after the administration of the administration preparation was used as a test sample. Fig. 3(2) is a graph showing a correlation between the dose ($\mu$mol/kg) and the $\Delta^{13}C$(‰) in the expired air in the case where the expired air collected 10 minutes after the administration of the administration preparation was used as a test sample. Fig. 3(3) is a graph showing a correlation between the dose ($\mu$mol/kg) and the $\Delta^{13}C$(‰) in the expired air in the case where the expired air collected 15 minutes after the administration of the administration preparation was used as a test sample.

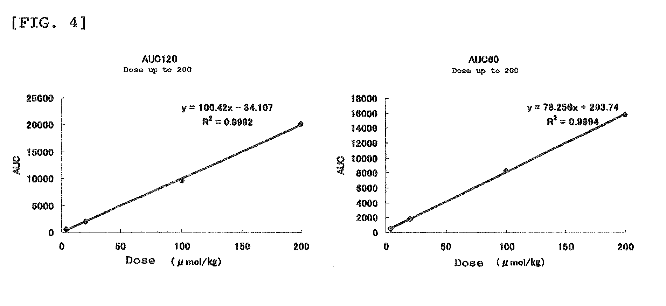

Fig. 4 shows correlations between the $^{13}C$-$CaCO_3$ dose ($\mu$mol/kg) of 4 to 200 $\mu$mol/kg and the "area under the $\Delta^{13}C$(‰)-time curve" (AUC) determined in Experimental Example 1. The graph of the AUC for an expired air collection time of 0 to 60 minutes (AUC60) versus the $^{13}C$-$CaCO_3$ dose (4 to 200 $\mu$mol/kg) is shown on the right side, whereas the graph of the AUC for an expired air collection time of 0 to 120 minutes (AUC120) versus the $^{13}C$-$CaCO_3$ dose (4 to 200 $\mu$mol/kg) is shown on the left side.

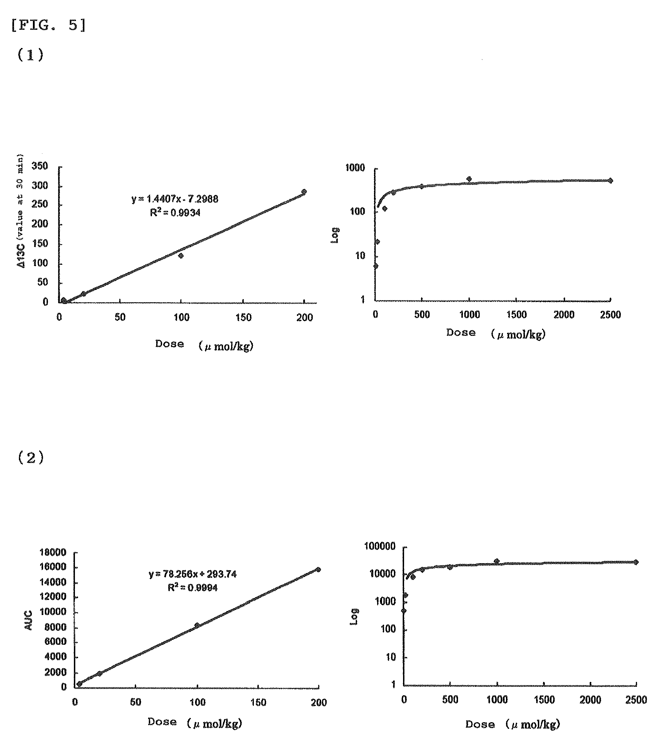

Fig. 5(1) shows, on the left side, a correlation between the $^{13}C$-$CaCO_3$ dose ($\mu$mol/kg) of 4 to 200 $\mu$mol/kg and the $\Delta^{13}C$(‰) at an expired air collection time of 30 minutes, which was determined in Experimental Example 1; and shows, on the right side, a relation between the $^{13}C$-$CaCO_3$ dose ($\mu$mol/kg) of 4 to 2500 $\mu$mol/kg and the $\Delta^{13}C$(‰) at an expired air collection time of 30 minutes, which was determined in Experimental Example 1 (the ordinate is indicated by Log). Fig. 5(2) shows, on the left side, a correlation between the $^{13}C$-$CaCO_3$ dose ($\mu$mol/kg) of 4 to 200 $\mu$mol/kg and the AUC for an expired air collection time of 0 to 60 minutes (AUC60), which was determined in Experimental Example 1 (this graph is the same as the graph shown on the right side of Fig. 4). Fig. 5(2) also shows a relation between the $^{13}C$-$CaCO_3$ dose ($\mu$mol/kg) of 4 to 2500 $\mu$mol/kg and the AUC60 on the right side (the ordinate is indicated by Log).

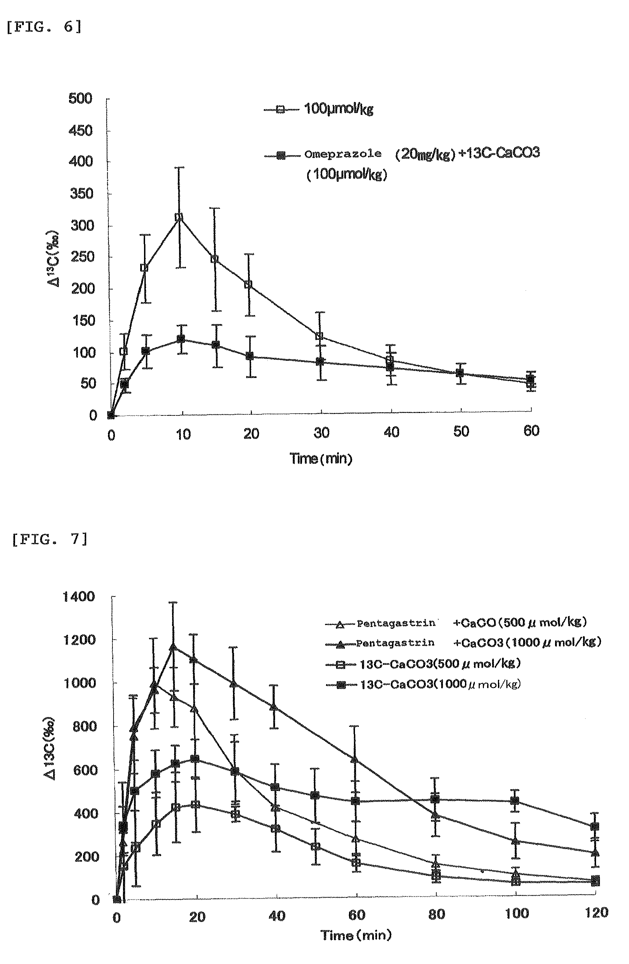

In Fig. 6, -□- and -■- respectively show changes in $\Delta^{13}C$(‰) ($\delta^{13}C_t$ in the expired air at each point in time for collecting the expired air (t) after the $^{13}C$-$CaCO_3$ administration minus $\delta^{13}C_0$ in the expired air before the $^{13}C$-$CaCO_3$ administration) in the expired air measured in Group 1 (control group: normal rats) and Group 2 (model group with decreased gastric acidity) versus the time lapsed after the administration of the $^{13}C$-$CaCO_3$ suspension (point in time for collecting the expired air) (Experimental Example 2).

In Fig. 7, -□- and -■- respectively show changes in $\Delta^{13}C$(‰) ($\delta^{13}C_t$ in the expired air at each point in time for collecting the expired air after the $^{13}C$-$CaCO_3$ administration minus $\delta^{13}C_0$ in the expired air before the $^{13}C$-$CaCO_3$ administration) in the expired air after administering the $^{13}C$-$CaCO_3$ suspension to Group 1 (control group: normal rats) at a dose of 500 $\mu$mol/kg and after administering the $^{13}C$-$CaCO_3$ suspension to Group 2 (control group: normal rats) at a dose of 1000 $\mu$mol/kg versus time (t) after the administration. Further, -△- and -▲- respectively show changes in $\Delta^{13}C$(‰) in the expired air after administering the $^{13}C$-$CaCO_3$ suspension to Group 3 (model group with increased gastric acidity) at a dose of 500 $\mu$mol/kg, and after administering the $^{13}C$-$CaCO_3$ suspension to Group 4 (model group with increased gastric acidity) at a dose of 1000 $\mu$mol/kg versus time (t) after the administration (Experimental Example 3).

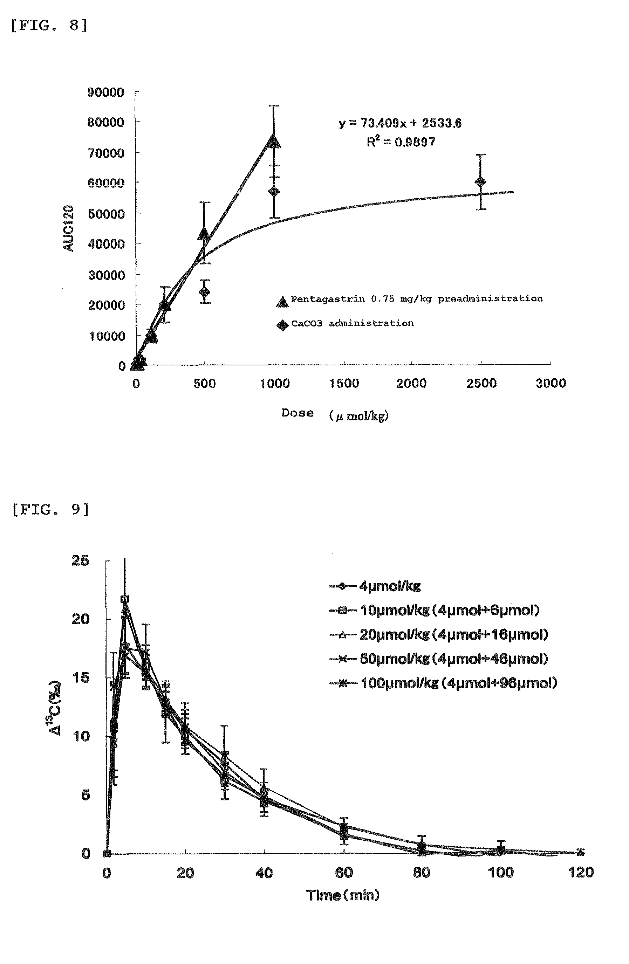

Fig. 8 shows correlations between the $^{13}C$-$CaCO_3$ dose ($\mu$mol/kg) of 4 to 1000 $\mu$mol/kg and the "area under the $\Delta^{13}C$(‰)-time curve" for an expired air collection time of 0 to 120 minutes (AUC120), which was determined in Experimental Example 3, in Group 1 (control group: normal rats, -♦-) and Group 4 (model group with increased gastric acidity, -▲-).

Fig. 9 shows changes in $\Delta^{13}C$(‰) ($\delta^{13}C_t$ in the expired air at each collection point in time (t) after the administration of the administration solution minus $\delta^{13}C_0$ in the expired air before the administration of the administration solution) in the expired air after administrating the $^{13}C$-$CaCO_3$ suspension at 4 $\mu$mol/4 mL to rats at a rate of 4 ml per kg body weight in Experimental Example 4, and after each of the administration solutions (10, 20, 50, and 100 $\mu$mol/4 mL) of a mixture of $^{12}C$-$CaCO_3$ at each concentration of 6, 16, 46, and 96 $\mu$mol/4 mL and $^{13}C$-$CaCO_3$ at 4 $\mu$mol/4 mL was administered to the rats at a rate of 4 ml per kg body weight in Experimental Example 4.

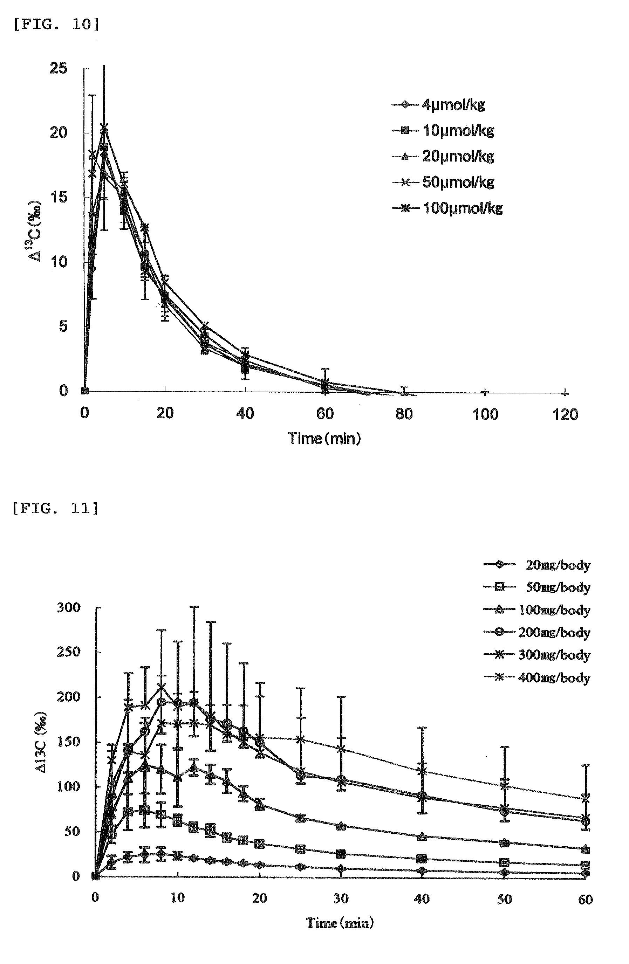

Fig. 10 shows changes in $\Delta^{13}C$(‰) ($\delta^{13}C$ value in the expired air at each collection point in time after administration minus $\delta^{13}C$ value in the expired air before administration) in the expired air after administering the $^{13}C$-$CaCO_3$

suspension at 4 μmol/4 mL to rats at a rate of 4 ml per kg body weight in Experimental Example 5 and after each of the mixtures (final concentrations: 10, 20, 50, and 100 μmol/4 mL) of $^{13}C$-$CaCO_3$ at 4 μmol/4 mL and sodium acetate was administered to the rats at a rate of 4 ml per kg body weight in Experimental Example 5.

Fig. 11 shows changes over time in $\Delta^{13}C$(‰) measured from $^{13}CO_2$ in the expired air after each of the $^{13}C$-$CaCO_3$ suspensions at various concentrations (400, 300, 200, 100, 50, and 20 mg/50 ml) was administered to humans (50 ml/body) in Experimental Example 6. In Fig. 11, the $\Delta^{13}C$(‰) in the expired air is plotted on the ordinate, and the expired air collection time (minutes) after the $^{13}C$-$CaCO_3$ administration is plotted on the abscissa.

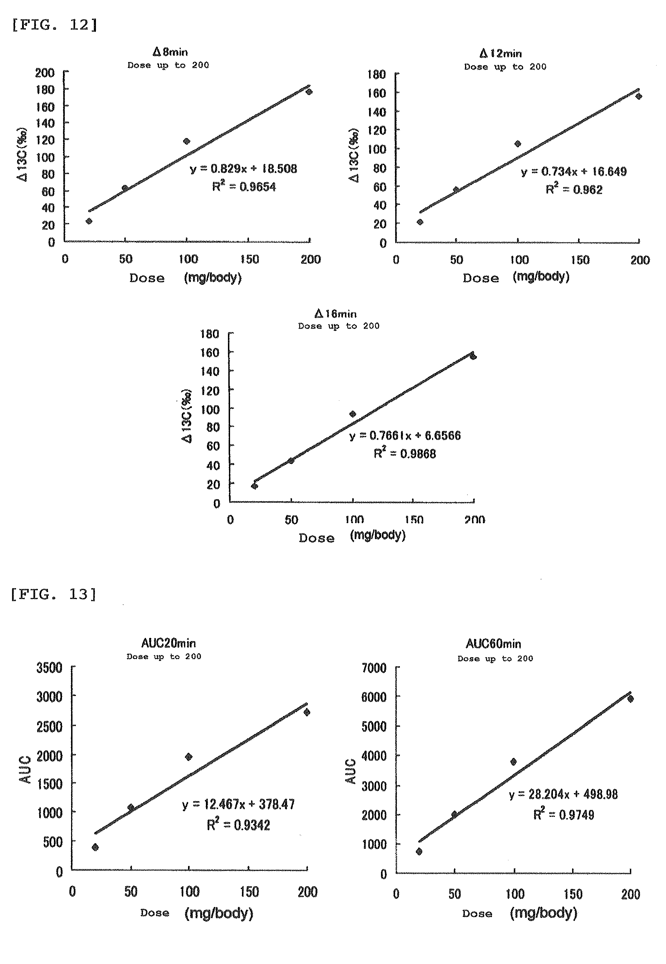

Fig. 12 shows correlations between the $^{13}C$-$CaCO_3$ dose (mg/body) of 20 to 200 mg/body and the $\Delta^{13}C$(‰) in the expired air in subjects (humans) (Experimental Example 6). Fig. 12(1) shows a correlation between the dose (mg/body) and the $\Delta^{13}C$(‰) in the expired air in the case where the expired air collected 8 minutes after the administration of the administration preparation was used as a test sample. Fig. 12(2) shows a correlation between the dose (mg/body) and the $\Delta^{13}C$(‰) in the expired air in the case where the expired air collected 12 minutes after the administration of the administration preparation was used as a test sample. Fig. 12(3) shows a correlation between the dose (mg/body) and the $\Delta^{13}C$(‰) in the expired air in the case where the expired air collected 16 minutes after the administration of the administration preparation was used as a test sample.

Fig. 13 shows correlations between the $^{13}C$-$CaCO_3$ dose (mg/kg) of 20 to 200 mg/body and the "area under the $\Delta^{13}C$(‰)-time curve" (AUC), which was determined in Experimental Example 6. The graph of the AUC for an expired air collection time of 0 to 20 minutes (AUC20) versus the $^{13}C$-$CaCO_3$ dose (20 to 200 mg/body) is shown on the left side, whereas the graph of the AUC for an expired air collection time of 0 to 60 minutes (AUC60) versus the $^{13}C$-$CaCO_3$ dose (20 to 200 mg/body) is shown on the right side.

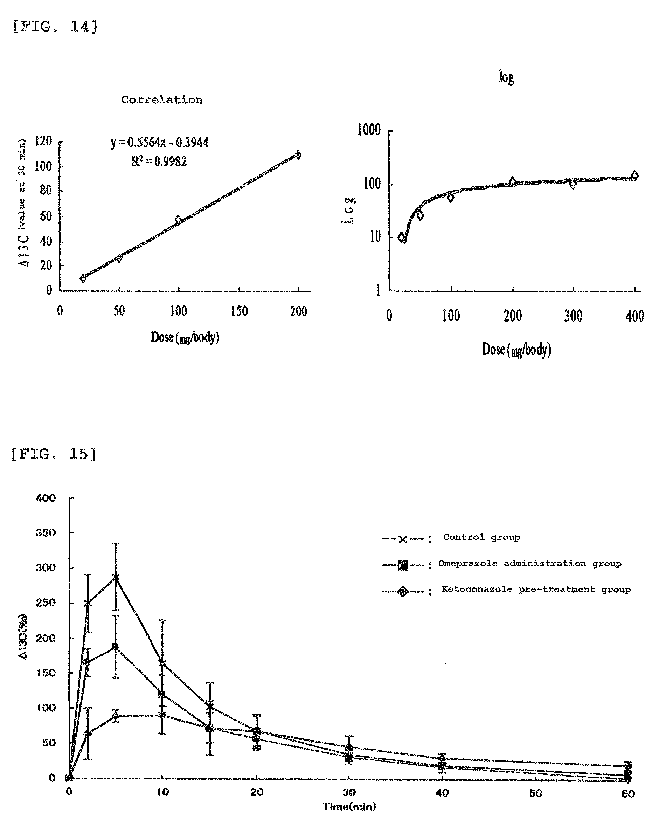

Fig. 14 shows, on the left side, a correlation between the $^{13}C$-$CaCO_3$ dose (mg/body) of 20 to 200 mg/body and the $\Delta^{13}C$(‰) at an expired air collection time of 30 minutes, which was determined in Experimental Example 6; and shows, on the right side, a relation between the $^{13}C$-$CaCO_3$ dose (mg/body) of 20 to 400 mg/body and the $\Delta^{13}C$(‰) at an expired air collection time of 30 minutes, which was determined in Experimental Example 6 (the ordinate is indicated by Log).

Fig. 15 is a graph showing the influence of CYP2C19 and CYP3A4 inhibitor ketoconazole on the gastric acid secretion ability of proton pump inhibitor omeprazole (see Experimental Example 7).

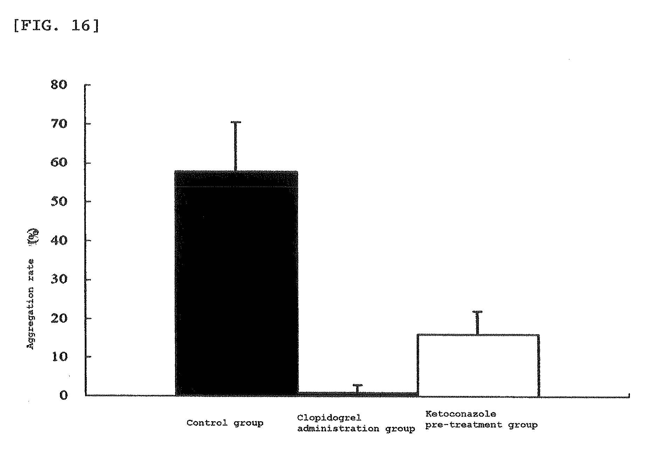

Fig. 16 is a graph showing the influence of CYP2C19 and CYP3A4 inhibitor ketoconazole on the platelet aggregation inhibitory action of clopidogrel (see Reference Example 1).

Description of Embodiments

(1) Preparation used for measuring gastric acidity

The method for measuring the gastric acidity of the present invention is performed, as described later, using, as a subject, a mammal to which a $^{13}C$-labeled carbonate compound is orally administered.

The $^{13}C$-labeled carbonate compound is not limited and may be any compound that, after being orally administered to a subject, reacts with gastric acid and in some cases is degraded or metabolized in the subject's stomach, and excreted in the expired air as $^{13}C$-labeled carbon dioxide ($^{13}CO_2$).

Examples of the compound that rapidly appears as $^{13}C$-labeled carbon dioxide in the expired air after being reacted with gastric acid in the stomach include a wide variety of $^{13}C$-labeled carbonate compounds that generate $^{13}C$-labeled carbonate ions ($^{13}CO_3^{-2}$) or $^{13}C$-labeled hydrogencarbonate ions ($H^{13}CO_3^{-1}$) in a molecule when dissolved. Examples of such a $^{13}C$-labeled carbonate compound include carbonate compounds in a narrow sense including alkali metal carbonates such as sodium carbonate and potassium carbonate; alkaline earth metal carbonates such as calcium carbonate, magnesium carbonate, and barium carbonate; and ammonium carbonate; and hydrogencarbonate compounds including alkali metal hydrogencarbonates such as potassium hydrogencarbonate and sodium hydrogencarbonate; and ammonium hydrogencarbonate. Preferable examples include calcium carbonate, magnesium carbonate, sodium carbonate, potassium carbonate, sodium hydrogencarbonate, and potassium hydrogencarbonate. Such compounds can correctly reflect and measure gastric acid output since they are unlikely to be influenced by physiological factors such as absorption and metabolism.

Examples of $^{13}C$-labeled compounds other than $^{13}C$-labeled carbonate compounds, which appear as $^{13}C$-labeled carbon dioxide ($^{13}CO_2$) in the expired air after being dissolved and then degraded or metabolized in the body, include $^{13}C$-labeled amino acids, proteins, organic acids, salts (e.g., alkali metal salts, such as Na) of organic acids, saccharides, lipids, and the like. These compounds generate $^{13}C$-labeled carbon dioxide in the expired air via the hepatic metabolism, after being digested and absorbed. Examples of the amino acids include glycine, phenylalanine, tryptophan, methionine, valine, histidine, and the like. Examples of the organic acids include acetic acid, lactic acid, pyruvic acid, butyric acid, propionic acid, octanoic acid, and their alkali metal salts. Examples of saccharides include glucose, galactose, xylose, lactose, and the like. Examples of the lipids include medium-chain triglycerides such as trioctanoin. However, these examples are not limitative. Preferably, an amino acid such as glycine, an organic acid such as acetic acid or octanoic acid, or an alkali metal salt (e.g., sodium salt or potassium salt) of such an organic acid can be used.

The method for labeling with the isotope ($^{13}$C) is not limited, and may be a conventional one. Further, a wide variety of known or commercially available $^{13}$C-labeled carbonate compounds, which is labeled with such an isotope ($^{13}$C), are usable (Sasaki, "5.1 Application of Stable Isotopes in Clinical Diagnosis": Kagaku no Ryoiki [Journal of Japanese Chemistry] 107, "Application of Stable Isotopes in Medicine, Pharmacy, and Biology", pp. 149-163 (1975), Nankodo: Kajiwara, RADIOISOTOPES, 41, 45-48 (1992), etc.).

The preparation to be administered to a subject (mammal) in the method of the present invention (hereinafter simply referred to as "administration preparation") may be the aforementioned $^{13}$C-labeled carbonate compound per se (used singly), or may be used in the form of a composition prepared by adding, as other ingredients, for example, an excipient such as lactose, sucrose, sodium chloride, glucose, urea, starch, calcium carbonate, kaolin, crystalline cellulose, or silicic acid; a binder such as simple syrup, glucose solution, starch solution, gelatin solution, carboxymethylcellulose, shellac, methylcellulose, potassium phosphate, or polyvinyl pyrrolidone; a disintegrator such as dry starch, sodium alginate, agar powder, laminaran powder, polyoxyethylene sorbitan fatty acid esters, sodium lauryl sulfate, stearic acid monoglyceride, starch, or lactose; an absorption accelerator such as quaternary ammonium base or sodium lauryl sulfate; a humectant, such as glycerin or starch; a lubricant such as purified talc, stearate, boric acid powder, or polyethylene glycol; other additives (for example, a flavor improver, taste improver, stabilizer, etc.); or the like.

The administration preparation used in the present invention may be a solid, semisolid, or liquid, and can be formulated into various forms such as powders, granules, tablets, pills, and liquids. However, it is preferable for the administration preparation used in the present invention to be dissolved promptly in the stomach. From this point of view, capsules in which a $^{13}$C-labeled compound is encapsulated with a capsule base are not particularly preferable. From the viewpoint of instant solubility as well, granules, tablets, and pills are not preferably covered by a pH-dependent soluble film or a poorly soluble sugarcoating.

The amount of the administration preparation is not limited, but can be suitably selected generally from the range of 10 mg to 20 g, and preferably from the range of 10 mg to 10 g, per unit dose because such an amount of the preparation is easy to take. The amount of the $^{13}$C-labeled carbonate compound contained per unit dose of the administration preparation is not limited, but can be suitably selected generally from the range of 10 mg to 5 g, preferably 10 mg to 4 g, more preferably 20 mg to 2 g per body. In addition, the amount can be suitably adjusted according to the type and individual body weight of a mammalian subject. The administration is performed within 30 minutes, preferably 20 minutes, and more preferably 15 minutes before the collection of the expired air for measuring gastric acidity, generally once or twice, preferably once.

(II) Method for measuring gastric acidity

The measurement of the gastric acidity of a mammal in the present invention is non-invasively performed using, as a test sample, the expired air collected from the mammal (hereinbelow sometimes referred to as a "subject") to which the aforementioned administration preparation ($^{13}$C-labeled carbonate compound or preparation containing the compound) has been orally administered.

The mammals targeted by the present invention are not limited as long as the respiratory system and digestive system (particularly gastric acid secretion system of the mammals has the same function as the respiratory system and digestive system of humans. Examples thereof include humans, monkeys, dogs, cats, rabbits, guinea pigs, rats, mice, and the like. Humans are preferably used; however, when test animals are used, dogs, rabbits, guinea pigs, rats, and mice are preferable because they are easily available and easy to handle.

A method including the following steps (1) to (3) is an embodiment of the method for measuring gastric acidity.

(1) The step in which using, as a test sample, the expired air of a mammalian subject excreted at any point in time within 30 minutes after oral administration of a predetermined amount of a $^{13}$C-labeled carbonate compound, the behavior of $^{13}CO_2$ excreted in the expired air is measured.

(2) The step in which the behavior of $^{13}CO_2$ (hereinbelow referred to as "measured $^{13}CO_2$ behavior") obtained in step (1) is compared with the behavior of the corresponding $^{13}CO_2$ (hereinbelow referred to as "reference $^{13}CO_2$ behavior") that has been obtained beforehand in a control mammal.

(3) The step in which the gastric acidity of the mammalian subject is determined based on the difference between the measured $^{13}CO_2$ behavior and the reference $^{13}CO_2$ behavior obtained above.

As the "$^{13}$C-labeled carbonate compound" used in steps (1) and (2) above, those mentioned in section (I) can be used. In place of the $^{13}$C-labeled carbonate compound, preparations containing the $^{13}$C-labeled carbonate compound explained in section (I) can be used. (In the present invention, a $^{13}$C-labeled carbonate compound and an oral admin-

istration preparation containing the $^{13}C$-labeled carbonate compound are correctively referred to as an "administration preparation.") In this sense, the "$^{13}C$-labeled carbonate compound" mentioned in step (1) above can also be referred to as the "administration preparation."

The respective steps in the method for measuring gastric acidity are explained below.

(1) Step of measuring the behavior of $^{13}CO_2$ excreted in the expired air

Step (1) is a step of collecting expired air from a subject (mammal) to which a predetermined amount of an administration preparation (a $^{13}C$-labeled carbonate compound or a preparation containing the compound) has been orally administered beforehand, and measuring the behavior of $^{13}CO_2$ contained in the collected expired air.

The method and the timing of orally administering the administration preparation to the subject are not limited. To measure basic gastric acidity (fasting gastric acidity), the preparation is preferably administered on an empty stomach to avoid the influence of foods. More preferably, it is desirable that the preparation be orally administered for 4 hours, even more preferably 10 hours after the start of fasting. However, to measure gastric acidity under general physiological conditions while reducing variations in the measured values between individuals, it is preferable to measure gastric acidity after stimulation of gastric acid secretion. In this case, it is preferable to administer a test beverage (e.g., water, beverage containing caffeine, alcoholic beverage, and consomme soup; and solid or liquid foods such as Calorie Mate (registered trademark)) or inject a gastric acid secretion stimulant (e.g., histamine hydrochloride, betazole hydrochloride, gastrin, insulin, etc.) to stimulate gastric acid secretion, and then orally administer a preparation at least one hour after the administration or injection.

When the administration preparation orally administered to the subject enters into the stomach, it dissolves with gastric juice to release a $^{13}C$-labeled carbonate compound, and the $^{13}C$-labeled carbonate compound is reacted with gastric acid to form $^{13}C$-labeled carbon dioxide ($^{13}CO_2$). The $^{13}C$-labeled carbon dioxide is gradually excreted in the expired air.

A reaction formula to form $^{13}C$-labeled carbon dioxide ($^{13}CO_2$) is shown below using $^{13}C$-labeled calcium carbonate ($Ca^{13}CO_3$) as the $^{13}C$-labeled carbonate compound.

$$Ca^{13}CO_3 + 2HCl \rightarrow CaCl_2 + H_2O + {}^{13}CO_2 \uparrow$$

The "behavior of $^{13}CO_2$" measured in the present invention is the following (ca) or (d):

(c) The difference $[\Delta^{13}C(‰) = \delta^{13}C_t - \delta^{13}C_0]$ (hereinbelow referred to as "$\Delta^{13}C(‰)_t$" or "$\Delta^{13}C(‰)$") between the "ratio of $^{13}CO_2$ amount to $^{12}CO_2$ amount" (hereinbelow referred to as "$\delta^{13}C_t$") included in the expired air collected at any point in time for collecting the expired air (t) within 30 minutes after the oral administration of a predetermined amount of the administration preparation, and the "ratio of $^{13}CO_2$ amount to $^{12}CO_2$ amount" (hereinbelow referred to as "$\delta^{13}C_0$") included in the expired air before the oral administration of the administration preparation.

(d) AUC "area under the $\Delta^{13}C(‰)$ -time curve" calculated by making a graph by plotting the time from the administration of the predetermined amount of the administration preparation to the collection of the expired air on the abscissa and the $\Delta^{13}C (‰)$ on the ordinate.

The behavior of $^{13}CO_2$ is $\Delta^{13}C(‰)$ or the "area under the $\Delta^{13}C(‰)$ time-curve" (AUC), preferably $\Delta^{13}C (‰)$.

Specifically, the behavior of such $^{13}CO_2$ can be measured as follows.

After the predetermined amount of the administration preparation is orally administered to a subject, the expired air is collected according to a conventional $^{13}C$ expiration test method (Kajiwara, RADIOISOTOPES, 41, 45-48 (1992); Kajiwara et al., RADIOISOTOPES, 41, 331-334 (1992), etc.). The amount of $^{13}CO_2$ excreted in the expired air is measured as the "$^{13}CO_2/^{12}CO_2$ concentration ratio ($\delta^{13}C$)" at each point in time for collecting the expired air (t) ("$\delta^{13}C_t$": the ratio of $^{13}CO_2$ amount to $^{12}CO_2$ amount (carbon dioxide) excreted in the expired air, t represents an expired air collection time (a time lapsed after the administration of the administration preparation)).

Subsequently, based on the difference between "$\delta^{13}C_t$" and the reference "$^{13}CO_2/^{12}CO_2$ concentration ratio ($\delta^{13}C$)" (hereinbelow sometimes referred to as "$\delta^{13}C_0$"), which has been measured beforehand prior to the administration of the administration preparation, "$\Delta^{13}C(‰)$" $[\Delta^{13}C(‰) = \delta^{13}C_t - \delta^{13}C_0]$ is calculated. The behavior of $^{13}CO_2$ excreted in the expired air at any point in time (t) within 30 minutes after the administration of the preparation can be obtained according to the above formula $[\Delta^{13}C(‰) = \delta^{13}C_t - \delta^{13}C_0]$ ("t" means an expired air collection time within 30 minutes from the administration of the administration preparation).

The behavior of $^{13}CO_2$ excreted in the expired air over time can be obtained by tracing the change of $\Delta^{13}C(‰)$ over time. Specifically, the behavior of $^{13}CO_2$ can be obtained by making a graph by plotting the expired air collection time (min); in other words, the lapse of time (min) after the administration of the preparation, on the abscissa and the $\Delta^{13}C(‰)$ on the ordinate.

The labeled substance ($^{13}CO_2$) contained in the collected expired air can be measured and analyzed by a conventional analysis technique, such as liquid scintillation counting, mass spectroscopy, infrared spectroscopic analysis, emission spectrochemical analysis, or magnetic resonance spectral analysis. From the viewpoint of measurement accuracy, infrared spectroscopic analysis and mass spectrometry are preferable.

Moreover, the "area under the $\Delta^{13}C$(‰)-time curve" (AUC) can be obtained by calculating the area under the curve based on the graph in which the lapse of time (min) after the administration of the administration preparation (expired air collection time: t) is plotted on the abscissa and the $\Delta^{13}C$(‰) is plotted on the ordinate.

The timing of expired air collection is generally at least 10 seconds and preferably at least one minute after the oral administration of the administration preparation. The time until the expired air is collected after the administration of the administration preparation is preferably within 30 minutes, more preferably 20 minutes, and even more preferably 15 minutes. For example, the expired air can be collected once or twice in the range of 10 seconds to 30 minutes, preferably 1 minute to 20 minutes, and more preferably 1 minute to 15 minutes after the oral administration of the administration preparation, and used as a test sample. The expired air is preferably collected once.

Fig. 1 (1) and (2) schematically illustrate the results of the thus-obtained $\Delta^{13}C$(‰) versus the dose of the administration preparation and the "area under the $\Delta^{13}C$(‰)-time curve" (AUC) versus the dose of the administration preparation, respectively. In Fig. 1, a shows the correlation between the dose and the $\Delta^{13}C$(‰) or AUC of a mammal having higher gastric acidity than normal; b shows the correlation between the dose and the $\Delta^{13}C$(‰) or AUC of a mammal having normal gastric acidity; and c shows a correlation between the dose and the $\Delta^{13}C$(‰) or AUC of a mammal having lower gastric acidity than normal.

As shown in the figure, regardless of the degree of gastric acidity, in all subjects having normal gastric acidity, subjects (mammals) having gastric acidity higher than the normal acidity (including, for example, patients with "hyperacidity"), and subjects (mammals) having gastric acidity lower than the normal acidity (including, for example, patients with "hypoacidity or anacidity," the correlation passing through the origin is seen between the dose of the administration preparation and $\Delta^{13}C$(‰), or between the dose and AUC up to a certain amount; however, the value becomes constant when the dose exceeds a certain amount. Such a certain amount is referred to as "$\Delta^{13}C$(‰) plateau value" or "AUC plateau value," and the dose at which the $\Delta^{13}C$(‰) or AUC reaches the plateau value is referred to as "reference dose."

The "$\Delta^{13}C$(‰) plateau value" and the "reference dose" vary depending on the subject's (mammalian) gastric acidity. Specifically, as shown in Fig. 1 (1), the "$\Delta^{13}C$(‰) plateau value" and the "reference dose" depend on the subject's (mammalian) gastric acidity. When the "$\Delta^{13}C$(‰) plateau value" and the "reference dose" of normal gastric acidity are regarded as standard values (hereinbelow referred to as "normal $\Delta^{13}C$(‰) plateau value" and "normal reference dose" for convenience in the present specification), a subject (subject with high gastric acidity) who has higher gastric acidity than normal, such as a patient with hyperacidity has a higher $\Delta^{13}C$(‰) plateau value than the normal $\Delta^{13}C$(‰) plateau value, and a higher reference dose than the normal reference dose. A subject (subject with low gastric acidity) who has lower gastric acidity than normal, such as a patient with hypoacidity or anacidity has a lower $\Delta^{13}C$(‰) plateau value than the normal $\Delta^{13}C$(‰) plateau value, and a lower reference dose than the normal reference dose.

The "$\Delta^{13}C$(‰) plateau value" and the "reference dose" of a subject (mammal) having higher gastric acidity than normal are referred to as "high-acidity $\Delta^{13}C$(‰) plateau value" and "high-acidity reference dose" for convenience. The "$\Delta^{13}C$(‰) plateau value" and the "reference dose" of a subject (mammal) having lower gastric acidity than normal are referred to as "low-acidity $\Delta^{13}C$(‰) plateau value" and "low-acidity reference dose" for convenience.

The "$\Delta^{13}C$(‰) plateau value" (normal $\Delta^{13}C$(‰) plateau value, high-acidity $\Delta^{13}C$(‰) plateau value, and low-acidity $\Delta^{13}C$(‰) plateau value) and the "reference dose" (normal reference dose, high-acidity reference dose, and low-acidity reference dose) can be obtained by performing the following steps (a) to (c) on a control mammal beforehand, and making a graph (hereinbelow referred to as a "dose-$\Delta^{13}C$(‰)" plot) in which the administration preparation dose is plotted on the abscissa and the $\Delta^{13}C$ (‰) is plotted on the ordinate.

(a) The step in which an administration preparation (including a preparation containing a $^{13}C$-labeled carbonate compound alone) is orally administered to a control mammal at a dose ranging from 0 to 2500 (dose), and the amount of $^{13}CO_2$ excreted in the expired air of the mammal is measured for each dose.

(b) The step in which $\Delta^{13}C$(‰)$_t$ is obtained by calculating a difference ($\Delta^{13}C$(‰) = $\delta^{13}C_t$-$\delta^{13}C_0$) between the ratio of $^{13}CO_2$ amount to $^{12}CO_2$ amount ($^{13}CO_2/^{12}CO_2$ concentration ratio) ("$\delta^{13}C_t$") in the expired air at a point in time for collecting the expired air (t) and the ratio of $^{13}CO_2$ amount to $^{12}CO_2$ amount ($^{13}CO_2/^{12}CO_2$ concentration ratio) ("$\delta^{13}C_0$") in the expired air before the administration, based on the amount of $^{13}CO_2$ obtained above.

(c) The step in which $\Delta^{13}C$(‰)$_t$ obtained in step (b) above is plotted against the dose of the administration preparation to form a calibration curve.

The control mammal used herein is preferably the same kind of animal as the mammalian subject. For example, when the measurement subject (mammal) is a human, the control subject is preferably a human (mammal); and when the measurement subject (mammal) is a rat, the control subject is preferably a rat (mammal). Although there is no limitation, the sex, age, weight, and the like of the control subject preferably correspond to those of the mammalian subject. In the above, the unit of dose (0 to 2500) is, for example, "$\mu$mol/kg" or "mg/body." As shown in the examples, when test animals such as rats are used, the unit "$\mu$mol/kg" can be used; and, for humans, the unit "mg/body" is preferably used.

The normal $\Delta^{13}C$(‰) plateau value and the normal reference dose can be calculated using a mammal (normal mammal) having normal gastric acidity as a control mammal. In general, common mammals have normal gastric acidity; however, for example, it may be better to confirm beforehand whether the control mammal has normal gastric acidity using the other measurement methods described, for example, in "Stomach, intestinal, pancreatic function examinations" (Outline of Clinical Examination, 33rd edition, Kanehara & Co., Ltd.) and Patent Literature 1. The gastric acidity used in the present invention corresponds to " [the hydrochloric acid concentration in the stomach (mEq/L)] x [gastric juice volume (L)] "

The high-acidity $\Delta^{13}C$(‰) plateau value and the high-acidity reference dose can be calculated using a mammal having higher gastric acidity than normal as a control mammal. Mammals having high gastric acidity can be distinguished by the other gastric acidity measurement methods (see Patent Literature 1 and the references mentioned above). Alternatively, mammals having high gastric acidity can be prepared using an artificial measure, i.e., administering to the normal mammal a drug that promotes and increases gastric acid secretion, as shown in Experimental Example 3 mentioned later, to increase gastric acidity. The thus-obtained mammals can be used.

The low-acidity $\Delta^{13}C$(‰) plateau value and the low-acidity reference dose can be calculated using a mammal having lower gastric acidity than normal as a control mammal. Mammals having low gastric acidity can be distinguished by the other gastric acidity measurement methods (see Patent Literature 1 and the references mentioned above). Alternatively, mammals having low gastric acidity can be prepared using an artificial measure, i.e., administering to the normal mammal a drug that inhibits gastric acid secretion, as shown in Experimental Example 2 mentioned later, to reduce gastric acidity. The thus-obtained mammals can be used.

In step (1), the dose of the administration preparation (a $^{13}C$-labeled carbonate compound or a preparation containing the compound) that has been administered beforehand to a subject (mammal) in step (1) can be determined based on the "dose-$\Delta^{13}C$(‰) plot" prepared for the control mammal beforehand, or the "reference dose" (normal reference dose, high-acidity reference dose, and low-acidity reference dose) obtained from the plot. In the present invention, this is called a "predetermined dose." The predetermined dose used in step (1) is generally lower than the high-acidity reference dose, preferably in the range of the low-acidity reference dose to the high-acidity reference dose, and more preferably the normal reference dose or approximately the normal reference dose (normal reference dose $\pm$ 100 $\mu$mol/kg or $\pm$ 100 mg/body). The normal reference dose is not limited, and may be about 200 $\mu$mol/kg or 200 mg/body.

Thus, the relation between the "$\Delta^{13}C$(‰) plateau value" and the "reference dose" is explained. As shown in Fig. 1, a similar relation can be seen between the "AUC plateau value" and the "reference dose"; therefore, the AUC plateau value can be used in place of the $\Delta^{13}C$(‰) plateau value.

(2) Step of comparing the measured $^{13}CO_2$ behavior and the reference $^{13}CO_2$ behavior

(3) Step of determining the gastric acidity of the mammalian subject

Step (2) is a step of comparing the behavior of $^{13}CO_2$ (measured $^{13}CO_2$ behavior) obtained in step (1) with the behavior of the corresponding $^{13}CO_2$ (reference $^{13}CO_2$ behavior) that has been obtained beforehand in a control mammal.

Step (3) is a step of determining the gastric acidity of a mammalian subject based on the difference between the measured $^{13}CO_2$ behavior and the reference $^{13}CO_2$ behavior obtained in step (2).

The "behavior of $^{13}CO_2$" is the following (c) or (d), and the behavior of $^{13}CO_2$ measured in the same kind of animal as the mammalian subject whose behavior of $^{13}CO_2$ (measured $^{13}CO_2$ behavior) is measured, is used as the reference $^{13}CO_2$ behavior.

(c) Difference [$\Delta^{13}C$ (‰) $=\delta^{13}C_t$-$\delta^{13}C_0$] between the "ratio of $^{13}CO_2$ amount to $^{12}CO_2$ amount" ($\delta^{13}C_t$) included in the expired air collected at any point in time (t) for collecting the expired air within 30 minutes after the oral administration of the predetermined amount of the administration preparation and the "ratio of $^{13}CO_2$ amount to $^{12}CO_2$ amount" ($\delta^{13}C_0$) included in the expired air before the oral administration of the administration preparation.

(d) AUC "area under the $\Delta^{13}C$(‰) -time curve" calculated by making a graph by plotting the time from the administration of the predetermined amount of the administration preparation to the collection of the expired air on the abscissa and the $\Delta^{13}C$ (‰) on the ordinate.

The behavior of $^{13}CO_2$ is $\Delta^{13}C$(‰) or AUC, and more preferably $\Delta^{13}C$(‰).

For example, when the $\Delta^{13}C$ (‰) is used as the "behavior of $^{13}CO_2$," the gastric acidity of the mammalian subject can be quantified by the following method.

(i) As a predetermined dose, the normal reference dose of the administration preparation is administered to a mammalian subject.

(ii) The expired air is collected at any point in time within 30 minutes after the administration, and the $\Delta^{13}C$ (‰) (this is called "measured $\Delta^{13}C$(‰)") is obtained based on the amount of $^{13}CO_2$ excreted in the expired air.

(iii) From the "dose-$\Delta^{13}C$(‰)" plot (calibration curve indicating the relation between the administration preparation dose and $\Delta^{13}C$ (‰)), which has been produced beforehand for the control mammal having normal gastric acidity, $\Delta^{13}C$(‰) (referred to as "reference $\Delta^{13}C$(‰)") corresponding to the normal reference dose is obtained, and compared with the measured $\Delta^{13}C$(‰).

(iv) When the measured $\Delta^{13}C$(‰) is lower than the reference $\Delta^{13}C$ (‰), the gastric acidity of the mammalian subject can be determined to be lower than the normal value.

(v) When the measured $\Delta^{13}C$(‰) is the same as the reference $\Delta^{13}C$ (‰), the gastric acidity of the mammalian subject can be determined to be the same as or higher than the normal value.

For example, with reference to the left figure of Fig. 12 (Experimental Example 6), which is the "dose-$\Delta^{13}C$(‰)" plot in which $Ca^{13}CO_3$ at a dose up to 200 mg/body is orally administered to a human, when the $\Delta^{13}C$(‰) is a value (140) that intersects with the "dose-$\Delta^{13}C$(‰)" plot at 8 minutes after the oral administration of 150 mg/body of the administration preparation ($Ca^{13}CO_3$), the gastric acidity is determined to be normal or higher than normal; and when the $\Delta^{13}C$(‰) is lower than that value (140), for example, 100, the gastric acidity is determined to be lower than normal.

In this case, the gastric acidity of the mammalian subject can be calculated as follows.

(a) Obtain the dose (mg/body or $\mu$mol/kg) (hereinbelow, referred to as "calcium carbonate equivalent amount") at which the "dose-$\Delta^{13}C$(‰)" plot intersects with the measured $\Delta^{13}C$(‰) value.

(b) Calculate the gastric acidity (M or Eq) using the calcium carbonate equivalent amount obtained in step (a) (mg/body or $\mu$mol/kg) according to the following formula.

```
Gastric acidity (mM[mmol]) = [calcium carbonate equivalent amount

(mg/body) x 2] / molecular weight of CaCO₃; or

Gastric acidity (μ Eq) = calcium carbonate equivalent amount

(μmol/kg) x weight of mammalian subject x 2
```

Based on the above, in the "dose-$\Delta^{13}C$(‰)" plot in the upper-left figure of Fig. 12, the calcium carbonate equivalent amount (mg/body) of the subject whose measured $\Delta^{13}C$ (‰) is 100 is determined to be 100 mg/body; therefore, the gastric acidity of the subject is 2 mM according to the following formula.

```
Gastric acidity (mM [mmol]) = [100 mg/body x 2]/100 (molecular

weight of CaCO₃).
```

As another method, when the "area under the $\Delta^{13}C$(‰)-time curve" (AUC) is used as the "behavior of $^{13}CO_2$," the gastric acidity of the mammalian subject can be quantified by the following method.

(i) As a predetermined dose, the normal reference dose of the administration preparation is administered to a mammalian subject.

(ii) After the administration, the expired air is collected over time, and the $\Delta^{13}C$ (‰) is obtained based on the amount of $^{13}CO_2$ excreted in the expired air at a predetermined period. The "area under the $\Delta^{13}C$(‰)-time curve" (AUC) (referred to as "measured AUC") is obtained by making a graph by plotting the time from the administration to the collection of the expired air on the abscissa and the $\Delta^{13}C$ (‰) on the ordinate.

(iii) From the "dose-AUC" plot (calibration curve indicating the relation between the dose of the administration preparation and the area under the $\Delta^{13}C$(‰)-time curve (AUC)), which has been produced beforehand for the control mammal, AUC (referred to as "reference AUC") corresponding to the normal reference dose is obtained, and compared with the measured AUC.

(iv) When the measured AUC is lower than the reference AUC, the gastric acidity of the mammalian subject can be determined to be lower than the normal value.

(v) When the measured AUC is the same as the reference AUC, the gastric acidity of the mammalian subject can be determined to be the same as or higher than the normal value.

In this case, similar to the case where the $\Delta^{13}C$(‰) is used as the "behavior of $^{13}CO_2$", the gastric acidity of the mammalian subject can be quantified as follows.

(a) Obtain the dose (mg/body or μmol/kg) (hereinbelow, referred to as "calcium carbonate equivalent amount") at which the "dose-AUC" plot intersects with the measured AUC value.

(b) Calculate the gastric acidity (M or Eq) using the calcium carbonate equivalent amount obtained in step (a) according to the following formula.

Gastric acidity (mM[mmol]) = [calcium carbonate equivalent amount (mg/body) x 2] / molecular weight of CaCO₃; or

Gastric acidity (μ Eq) = calcium carbonate equivalent amount (μmol/kg) x weight of mammalian subject x 2

The method for measuring the gastric acidity of the present invention explained above can be used as a method for diagnosing or evaluating the presence or absence of a decrease or increase of the basic gastric acid secretion of mammals.

The method for measuring the gastric acidity of the present invention, particularly, the method using $\Delta^{13}C$(‰) as the "$^{13}CO_2$ behavior" is useful in that the method can non-invasively evaluate and diagnose the gastric acidity in a simple manner with few expired-air collections (preferably one time), and without restraining a subject over a long period of time. By using the measurement method of the gastric acidity of the present invention, gastric acidity tendency (gastric hyperacidity, normal, hypoacidity, anacidity, etc.) can be measured, and consequently, diseases involving gastric acid secretion can be diagnosed. Further, the drug effect or treatment effect of the gastric acid reducer can be evaluated. Specifically, the evaluation can be performed by measuring the gastric acidity before and after the administration of the gastric acid reducer to a subject using the administration preparation of the present invention, and comparing the results. By this method, the drug effect of the administration drug (gastric acid reducer), and the treatment effect of each drug on the subject can be evaluated. Consequently, the method can be used as a means for selecting a drug (gastric acid reducer) suitable for each subject.

In addition, as a gastric acid reducer, drugs having an effect of increasing gastric pH, including gastric acid secretion inhibitors such as proton pump inhibitors (PPI) and $H_2$ blockers, and drugs having an effect of neutralizing gastric acid such as antacids can be used. Therefore, the present invention can be used as a method for detecting a gastric pH change by the drugs to evaluate the effect of the drugs on a subject.

To perform the method of the present invention, when the subject's gastric acidity is predicted to be high beforehand, the gastric acidity of the subject can be accurately or economically evaluated by using the high-acidity reference dose as a predetermined dose. When the effect of the gastric acid reducer on a subject having high gastric acidity is evaluated, since a decrease in the gastric acidity due to the administration of such a drug is anticipated, the normal reference dose is used as a predetermined dose, and the effect of the drug can thereby be accurately or economically evaluated. When the subject's gastric acidity is predicted to be low beforehand, the gastric acidity of the subject can be economically evaluated by using the low acid reference dose as a predetermined dose.

Of the gastric acid reducers, proton pump inhibitors such as omeprazole and lansoprazole are drugs metabolized by hepatic metabolism enzymes such as CYP2C19 and CYP3A4. Therefore, the effects of these drugs depend on the enzyme activity (metabolic activity) of CYP2C19 and CYP3A4 in a subject; however, since these enzymes have a plural of genetic polymorphism, and the enzyme activity (metabolic capacity) is different depending on the genetic polymorphism, the effect of omeprazole and that of lansoprazole are different. By using the gastric acidity measurement method of the present invention, the enzyme activity (metabolic activity) of CYP2C19 and CYP3A4 in a subject can be measured and evaluated based on the gastric acidity after the administration of omeprazole or lansoprazole. Even when drugs other than omeprazole and lansoprazole are used, by evaluating the gastric acidity after the administration of omeprazole or lansoprazole, the susceptibility of a subject to drugs whose activity is eliminated by being metabolized by CYP2C19 and CYP3A4, or drugs that exhibit activity by being metabolized by such enzymes (e.g., Plavix) can also be evaluated.

(III) Method for measuring the effect of a gastric acid reducer

As indicated above, by using the gastric acidity measurement method of the present invention, the effect of the gastric acid reducer on a mammalian subject can be measured. Specifically, the effect-measuring method can be performed by the following steps (1) to (4).
(1) The step in which using, as a test sample, the expired air of a mammalian subject excreted at any point in time within 30 minutes after oral administration of a predetermined amount of a $^{13}C$-labeled carbonate compound, the oral administration being performed after administration of a gastric acid reducer, the behavior of $^{13}CO_2$ excreted in the expired air is measured.
(2) The step in which the behavior of $^{13}CO_2$ (measured $^{13}CO_2$ behavior) obtained in step (1) is compared with the behavior of corresponding $^{13}CO_2$ (reference $^{13}CO_2$ behavior) measured in a mammal (control mammal) to which a predetermined amount of a $^{13}C$-labeled carbonate compound has been orally administered beforehand without administering the gastric acid reducer.
(3) The step in which the gastric acidity of the mammalian subject is determined based on the difference between the reference $^{13}CO_2$ behavior and the measured $^{13}CO_2$ behavior obtained above.
(4) The step in which the effect of the gastric acid reducer on the mammalian subject is determined using the gastric acidity of the mammalian subject obtained above as an index.

The gastric acid reducer includes a proton pump inhibitor and an $H_2$ blocker each having an effect of inhibiting gastric acid secretion, and an antacid having an effect of neutralizing gastric acid.
A proton pump inhibitor is a drug that acts on proton pump of gastric parietal cells, and inhibits gastric acid secretion. Examples of the drugs include omeprazole, lansoprazole, pantoprazole, rabeprazole, esomeprazole, and the like. An $H_2$ blocker is a drug that inhibits gastric acid secretion by competitively antagonizing a histamine $H_2$ receptor, which is present in gastric parietal cells and enhances gastric acid secretion. Examples thereof include ranitidine, cimetidine, famotidine, nizatidine, lafutidine, roxatidine acetate hydrochloride, and the like. Further, an antacid is a drug that has an effect of neutralizing overproduced gastric acid to adjust gastric pH. Examples thereof include magnesium hydroxide, anhydrous dibasic calcium phosphate, precipitated calcium carbonate, sodium hydrogencarbonate, magnesium oxide, and the like.
Step (1), in which the behavior of $^{13}CO_2$ excreted in the expired air is measured, can be performed on a mammalian subject to which a $^{13}C$-labeled carbonate compound is orally administered after the administration of a gastric acid reducer whose effect is an interest of evaluation. The mammalian subject is preferably a human, as in the method for measuring gastric acidity. Test animals such as monkeys, dogs, cats, rabbits, guinea pigs, rats, mice, etc., can also be used.
The interval between the administration of the gastric acid reducer and the administration of the $^{13}C$-labeled carbonate compound to a mammalian subject is not limited, but is generally in the range of 1 minute to 12 hours, preferably 2 to 480 minutes, and more preferably 5 to 240 minutes.
Except for the use of the mammalian subject to which a gastric acid reducer is administered before the administration of a $^{13}C$-labeled carbonate compound, step (1) is performed in the same manner as in the method explained in Item (1) of the section "(II) Method for measuring gastric acidity" above. Similarly, as the $^{13}C$-labeled carbonate compound, the administration preparation explained in the section "(I) Preparation used for measuring gastric acidity" can be used.
Step (2), in which the measured $^{13}CO_2$ behavior and the reference $^{13}CO_2$ behavior are compared, is a step of comparing the $^{13}CO_2$ behavior (measured $^{13}CO_2$ behavior) obtained in step (1) and the corresponding $^{13}CO_2$ behavior (reference $^{13}CO_2$ behavior) that has been obtained beforehand in a control mammal. The control mammal used herein is a mammal to which a predetermined amount of a $^{13}C$-labeled carbonate compound is orally administered as in the mammalian subject, without administering a gastric acid reducer, and is generally the same kind of mammalian subject, as explained in Item (II) above. There is no particular limitation, but the control mammal preferably has the same sex, and almost the same age and weight as the mammalian subject.
Except for the use of the $^{13}CO_2$ behavior obtained in the above-mentioned control mammal as the reference $^{13}CO_2$ behavior, step (2) can use the same method explained in Items (1) and (2) of the section "(II) Method for measuring gastric acidity."
Step (3), which determines the gastric acidity, can be performed based on a difference between the reference $^{13}CO_2$ behavior and the measured $^{13}CO_2$ behavior obtained in step (2), and can use the same method explained in Items (2) and (3) of the section "(II) Method for measuring gastric acidity."
Step (4), which determines the effect of the gastric acid reducer, is carried out using the gastric acidity of the mammalian subject obtained in step (3) as an index.

Specifically, in step (4), when the gastric acidity of the mammalian subject measured in step (3) is the same as or higher than the gastric acidity of the control mammal, the administered gastric acid reducer can be determined as having no effect on the mammalian subject. When the gastric acidity of the mammalian subject measured in step (3) is lower than the gastric acidity of the control mammal, the administered gastric acid reducer can be determined as having an effect on a mammalian subject.

(IV) Method for evaluating the enzyme activity (metabolic capacity) of CYP2C19 alone or both CYP2C19 and CYP3A4 in a mammalian subject, effect of a drug metabolized by CYP2C19 alone or both CYP2C19 and CYP3A4 on a mammalian subject, or/and susceptibility of a mammalian subject to the drug

As indicated above, using the gastric acidity measurement method of the present invention, the enzyme activity (metabolic capacity) of CYP2C19 alone or both CYP2C19 and CYP3A4 in the mammalian subject, effect metabolized by CYP2C19 alone or both CYP2C19 and CYP3A4 on the mammalian subject, or/and susceptibility of the mammalian subject to the drug can be evaluated.

Specifically, the evaluation method can be carried out by performing the following steps (1) to (4).

(1) The step in which using, as a test sample, the expired air of a mammalian subject excreted at any point in time within 30 minutes after oral administration of a predetermined amount of a $^{13}C$-labeled carbonate compound, the oral administration being performed after administration of omeprazole or lansoprazole, the behavior of $^{13}CO_2$ excreted in the expired air is measured.

(2) The step in which the behavior of $^{13}CO_2$ (measured $^{13}CO_2$ behavior) obtained in step (1) is measured with the behavior of corresponding $^{13}CO_2$ (reference $^{13}CO_2$ behavior) measured in a mammal (control mammal) to which a predetermined amount of a $^{13}C$-labeled carbonate compound has been orally administered beforehand without administering the omeprazole or lansoprazole.

(3) The step in which the gastric acidity of the mammalian subject is determined based on the difference between the reference $^{13}CO_2$ behavior and the measured $^{13}CO_2$ behavior obtained above.

(4) The step in which the enzyme activity (metabolic capacity) of CYP2C19 alone or both CYP2C19 and CYP3A4 in the mammalian subject, effect metabolized by CYP2C19 alone or both CYP2C19 and CYP3A4 on the mammalian subject, or/and susceptibility of the mammalian subject to the drug are determined using the gastric acidity of the mammalian subject obtained above as an index.

Here, omeprazole and lansoprazole are both drugs that are metabolized in the body by the effects of CYP2C19 and CYP3A4, which are hepatic metabolism enzymes.

Step (1), in which the behavior of $^{13}CO_2$ excreted in the expired air is measured, can be performed on a mammalian subject to which a $^{13}C$-labeled carbonate compound is orally administered after the administration of omeprazole or lansoprazole. The mammalian subject is preferably a human, as in the method for measuring gastric acidity. Test animals such as monkeys, dogs, cats, rabbits, guinea pigs, rats, mice, etc., can also be used.

The interval between the administration of omeprazole or lansoprazole and the administration of the $^{13}C$-labeled carbonate compound to the mammalian subject is not limited, but it is generally in the range of 1 minute to 12 hours, preferably 2 to 480 minutes, and more preferably 5 to 240 minutes.

Except for the use of the mammalian subject to which omeprazole or lansoprazole is administered before the administration of a $^{13}C$-labeled carbonate compound, step (1) is performed in the same manner as in the method explained in Item (1) of the section "(II) Method for measuring gastric acidity" above. Similarly, as the $^{13}C$-labeled carbonate compound, the administration preparation explained in the section "(I) Preparation used for measuring gastric acidity" can be used.

Step (2) in which the measured $^{13}CO_2$ behavior and the reference $^{13}CO_2$ behavior are compared is a step of comparing the $^{13}CO_2$ behavior (measured $^{13}CO_2$ behavior) obtained in step (1) and the corresponding $^{13}CO_2$ behavior (reference $^{13}CO_2$ behavior) that has been obtained beforehand in a control mammal. The control mammal used herein is a mammal to which a predetermined amount of a $^{13}C$-labeled carbonate compound is orally administered as in the mammalian subject, without administering omeprazole or lansoprazole; and, as explained in Item (II) above, the control mammal is generally the same kind of the mammalian subject. There is no particular limitation, but the control mammal preferably has the same sex, and almost the same age and weight as the mammalian subject.

Except for the use of the $^{13}CO_2$ behavior obtained in the above-mentioned control mammal as the reference $^{13}CO_2$ behavior, step (2) can use the same method as explained in Items (1) and (2) of the section "(II) Method for measuring gastric acidity."

Step (3) that determines the gastric acidity can be performed based on a difference between the reference $^{13}CO_2$ behavior obtained in step (2) and the measured $^{13}CO_2$ behavior, and can use the same method as explained in Items (2) and (3) of the section "(II) Method for measuring gastric acidity."

Using the gastric acidity of the mammalian subject obtained in step (3) as an index, step (4), i.e., (a) determining the enzyme activity (metabolic capacity) of CYP2C19 alone or both CYP2C19 and CYP3A4 in the mammalian subject, (b) determining the effect of the drug metabolized by CYP2C19 alone or both CYP2C19 and CYP3A4, or/and (c) determining the susceptibility of the mammalian subject to the drug can be performed.

Step (4) may be a step of determining any one of or at least two items of above (a) to (c) relating to the mammalian subject, using the gastric acidity of the mammalian subject as an index. Preferably, it is the step of determining the enzyme activity (metabolic capacity) of CYP2C19 alone or both CYP2C19 and CYP3A4 in the mammalian subject (step (a)), the step of determining the effect of a drug metabolized by CYP2C19 alone or both CYP2C19 and CYP3A4 (step (b)), and the step of performing step (b) after step (a).

In the case where step (4) includes step (a) of determining the enzyme activity (metabolic capacity) of CYP2C19 alone or both CYP2C19 and CYP3A4 in a subject mammal, when the gastric acidity of the mammalian subject measured in step (3) is the same as or higher than that of the control mammal, step (4) can determine that the enzyme activity (metabolic capacity) of CYP2C19 alone or both CYP2C19 and CYP3A4 in the mammalian subject is normal or higher than normal. Alternatively, when the gastric acidity of the mammalian subject measured in step (3) is lower than that of the control mammal, step (4) can determine that the enzyme activity (metabolic capacity) of CYP2C19 alone or both CYP2C19 and CYP3A4 in the mammalian subject is lower than normal.

In the case where step (4) includes step (b) of determining the effect of the drug metabolized by CYP2C19 alone or both CYP2C19 and CYP3A4, the effect of the drug is different when the drug loses or reduces its activity when metabolized by CYP2C19 alone or both CYP2C19 and CYP3A4, and when the drug expresses or increases its activity when metabolized by CYP2C19 alone or both CYP2C19 and CYP3A4.

In the case where the drug that loses or reduces its activity when metabolized by CYP2C19 alone or both CYP2C19 and CYP3A4 is used as a drug, when the gastric acidity of the mammalian subject measured in step (3) is the same as or higher than that of the control mammal, step (4) can determine that the effect of the drug on the subject is low; and when the gastric acidity of the mammalian subject measured in step (3) is lower than that of the control mammal, step (4) can determine that the effect of the drug on the subject is high.

On the other hand, in the case where the drug that expresses or increases its activity when metabolized by CYP2C19 alone or both CYP2C19 and CYP3A4 is used as a drug, when the gastric acidity of the mammalian subject measured in step (3) is the same as or higher than that of the control mammal, step (4) can determine that the effect of the drug on the mammalian subject is high; and when the gastric acidity of the mammalian subject measured in step (3) is lower than that of the control mammal, step (4) can determine that the effect of the drug on the subject is low.

In the case where step (4) includes step (c) of determining the susceptibility of the mammalian subject to the drug metabolized by CYP2C19 alone or both CYP2C19 and CYP3A4, the susceptibility is different when the drug loses or reduces its activity when metabolized by CYP2C19 alone or both CYP2C19 and CYP3A4, and when the drug expresses or increases its activity when metabolized by CYP2C19 alone or both CYP2C19 and CYP3A4.

In the case where the drug that loses or reduces its activity when metabolized by CYP2C19 alone or both CYP2C19 and CYP3A4 is used as a drug, when the gastric acidity of the mammalian subject measured in step (3) is the same as or higher than that of the control mammal, step (4) can determine that the susceptibility of the mammalian subject to the drug is low; and when the gastric acidity of the mammalian subject measured in step (3) is lower than that of the control mammal, step (4) can determine that the susceptibility of the mammalian subject to the drug is high.

In the case where the drug that expresses or increases its activity when metabolized by CYP2C19 alone or both CYP2C19 and CYP3A4 is used as a drug, when the gastric acidity of the mammalian subject measured in step (3) is the same as or higher than that of the control mammal, step (4) can determine that the susceptibility of the mammalian subject to the drug is high; and when the gastric acidity of the mammalian subject measured in step (3) is lower than that of the control mammal, step (4) can determine that the susceptibility of the drug to the subject is low.

Examples

The following experimental examples are provided to illustrate the present invention, and are not to limit the scope of the present invention.

Experimental Example 1

Evaluation of a correlation between the dose ($\mu$mol/kg) of $^{13}$C-CaCO$_3$ and the difference between $^{13}$CO$_2$/$^{12}$CO$_2$ concentration ratios ($\delta^{13}$ values) before and after administration [$\Delta^{13}$C(‰) = ($\delta^{13}$ value)$_t$ - ($\delta^{13}$ value)$_0$], and evaluation of a correlation between the dose ($\mu$mol/kg) of $^{13}$C-CaCO$_3$ and the "area under the $\Delta^{13}$C(‰) -time curve" (AUC)

(1) Preparation of $^{13}$C-CaCO$_3$ administration solutions

A $^{13}$C-CaCO$_3$ suspension (15 mL) at a concentration of 2500 $\mu$mol/4 mL was prepared by adding, in small portions, a 0.5% CMC aqueous solution (sodium carboxymethyl cellulose dissolved in distilled water for injection) to 946.8 mg of $^{13}$C-CaCO$_3$ (MW:101, manufactured by Cambridge Isotope Laboratory) while kneading the resulting mixture in a mortar. The obtained $^{13}$C-CaCO$_3$ suspension was diluted with the 0.5% CMC aqueous solution to prepare administration solutions at various concentrations (2500, 1000, 500, 200, 100, 20, and 4 $\mu$mol/4 mL).

(2) Experiment

Each of the $^{13}$C-CaCO$_3$ suspensions at various concentrations prepared above was forcibly administered orally (4 ml/kg) to fasted rats (male, SD rats: n=3) as experimental animals. Expired air was collected before the administration of the $^{13}$C-CaCO$_3$ suspensions (before the $^{13}$C-CaCO$_3$ administration) (0 minute) and at each point in time (2, 5, 10, 15, 20, 30, 40, 50, 60, 80, 100, and 120 minutes) after the administration of the $^{13}$C-CaCO$_3$ suspensions (after the $^{13}$C-CaCO$_3$ administration), and $\Delta^{13}$C(‰) was determined with a mass spectrometer for expired air analysis (ABCA: manufactured by SerCon) from the concentration of $^{13}$CO$_2$ excreted in the expired air. The $\Delta^{13}$C(‰) was determined by measuring $^{13}$CO$_2$/$^{12}$CO$_2$ concentration ratios ($\delta^{13}$C values) in the expired air before the $^{13}$C-CaCO$_3$ administration (0 minute) and in the expired air at each point in time for collecting the expired air (t minutes) after the $^{13}$C-CaCO$_3$ administration, and calculating $\Delta^{13}$C(‰) from the difference [($\delta^{13}$C value)$_t$ - ($\delta^{13}$C value)$_0$] between the $\delta^{13}$C value [($\delta^{13}$C value)$_t$] at each collection point in time (t) after the $^{13}$C-CaCO$_3$ administration and the $\delta^{13}$C value [($\delta^{13}$C value)$_0$] before the $^{13}$C-CaCO$_3$ administration (the same applies to the below-described experimental examples).

Fig. 2 shows changes in the $\Delta^{13}$C(‰) in the expired air measured after each of the $^{13}$C-CaCO$_3$ suspensions at various concentrations was administered to the rats. In Fig. 2, the $\Delta^{13}$C(‰) in the expired air is plotted on the ordinate, and the expired air collection time (minutes) after the $^{13}$C-CaCO$_3$ administration is plotted on the abscissa. As shown in Fig. 2, it was observed that the $^{13}$C-CaCO$_3$ was metabolized in the body and excreted as $^{13}$CO$_2$ in the expired air. In addition, a saturation phenomenon was observed when the dose exceeded 200 $\mu$mol/kg (-■-).

Fig. 3 shows relations between the $^{13}$C-CaCO$_3$ suspension dose ($\mu$mol/kg) of 4 $\mu$mol/kg to 200 $\mu$mol/kg and the $\Delta^{13}$C(‰) in the expired air at each point in time for collecting the expired air ((1) 5 minutes after the administration, (2) 10 minutes after the administration, and (3) 15 minutes after the administration). Fig. 3 reveals that the $\Delta^{13}$C(‰) in the expired air versus the dose ($\mu$mol/kg) up to 200 $\mu$mol/kg shows a straight line nearly passing through the origin in all expired air samples, regardless of when the expired air was collected. That is, when the expired air collected at least 5 minutes after the administration of the administration preparation in the dose range of at least not greater than 200 $\mu$mol/kg was used as a test sample, a linear correlation was observed between the dose ($\mu$mol/kg) of the $^{13}$C-CaCO$_3$ and the $\Delta^{13}$C (‰) in the expired air (R$^2$ values: 0.9 or more).

Fig. 4 shows correlations between the $^{13}$C-CaCO$_3$ suspension dose ($\mu$mol/kg) of 4 $\mu$mol/kg to 200 $\mu$mol/kg and the "area under the $\Delta^{13}$C(‰)-expired air collection time (60 minutes or 120 minutes) curve" (AUC). The graph of the AUC for an expired air collection time of 0 to 60 minutes (AUC60) versus the $^{13}$C-CaCO$_3$ dose (4 to 200 $\mu$mol/kg) is shown on the right side, whereas the graph of the AUC for an expired air collection time of 0 to 120 minutes (AUC120) versus the $^{13}$C-CaCO$_3$ dose (4 to 200 $\mu$mol/kg) is shown on the left side. As is clear from these graphs, the AUCs for an expired air collection time of 0 to 60 minutes and for an expired air collection time of 0 to 120 minutes (AUC60 and AUC120) versus the $^{13}$C-CaCO$_3$ dose ($\mu$mol/kg) up to 200 $\mu$mol/kg show straight lines nearly passing through the origin. That is, linear correlations were observed between the AUCs and the $^{13}$C-CaCO$_3$ dose in the range of at least not greater than 200 $\mu$mol/kg.

The above results indicate that in mammals with normal gastric acidity, there is a linear correlation between the dose of the $^{13}$C-labeled carbonate compound in the range of not greater than 200 $\mu$mol/kg, and the $^{13}$CO$_2$ excretion behavior in the expired air ($^{13}$CO$_2$ concentration ($\Delta^{13}$C(‰)) in the expired air at each point in time for collecting the expired air and the "area under the $\Delta^{13}$C(‰)-time curve" (AUC)).

As shown in Figs. 3 and 4, there is a linear correlation between the $^{13}$C-CaCO$_3$ suspension dose ($\mu$mol/kg) up to 200 $\mu$mol/kg and the $\Delta^{13}$C (‰), and there is a linear correlation between the $^{13}$C-CaCO$_3$ suspension dose ($\mu$mol/kg) up to 200 $\mu$mol/kg and the "area under the $\Delta^{13}$C(‰)-time curve" (AUC). Fig. 5 (1) shows a relation between the dose of 4 to 2500 $\mu$mol/kg and the $\Delta^{13}$C (‰) at an expired air collection time of 30 minutes. In Fig. 5(1), a relation between the $^{13}$C-CaCO$_3$ dose (4 to 200 $\mu$mol/kg) and the $\Delta^{13}$C (‰) is shown on the left side, whereas the relation between the

$^{13}C$-$CaCO_3$ dose (4 to 2500 $\mu$mol/kg) and the $\Delta^{13}C$(‰) is shown on the right side. The results reveal that when the $^{13}C$-$CaCO_3$ dose is not greater than a certain amount, there is a linear correlation between the dose and the $\Delta^{13}C$ (‰); however, when the dose exceeds the certain amount, the $\Delta^{13}C$(‰) value becomes constant (plateau), as shown in Fig. 1 (1).

Fig. 5 (2) shows a relation between the dose of 4 to 2500 $\mu$mol/kg and AUC for an expired air collection time of 0 to 60 minutes. In Fig. 5 (2), a relation between the $^{13}C$-$CaCO_3$ dose (4 to 200 $\mu$mol/kg) and the AUC is shown on the left side (the same as the graph on the right side of Fig. 4), whereas the relation between the $^{13}C$-$CaCO_3$ dose (4 to 2500 $\mu$mol/kg) and the AUC is shown on the right side. The results reveal that as in the above $\Delta^{13}C$ (‰), when the $^{13}C$-$CaCO_3$ dose is not greater than a certain amount, there is a linear correlation between the dose and the AUC; however, when the dose exceeds the certain amount, the AUC value becomes constant (plateau), as shown in Fig. 1 (2).

Experimental Example 2

Measurement of gastric acid secretion decrease

(1) Preparation of a proton pump inhibitor (PPI: omeprazole) administration solution

To 1 vial of Omepral Injection 20 (omeprazole 20 mg/vial, AstraZeneca K.K.) that has the action of suppressing gastric acid secretion, 1 mL of physiological saline was added so that the concentration was 20 mg/mL, and the resulting mixture was used as a PPI administration solution.

(2) Experiment

Fasted rats (male, SD rats) were used as experimental animals.

To Group 1 (control group: normal rats, n=3), the $^{13}C$-$CaCO_3$ suspension (100 $\mu$mol/4 mL) prepared in Experimental Example 1 was orally administered (4 mL/kg). To Group 2 (model group with decreased gastric acidity: rats given PPI, n=3), the PPI administration solution (20 mg/mL) was intravenously administered (1 mL/kg) to produce animal models with suppressed gastric acid secretion, and the $^{13}C$-$CaCO_3$ suspension (100 $\mu$mol/4 mL) was orally administered (4 mL/kg) 4 hours after the administration of the PPI administration solution.

In the rats in each group, expired air was collected before the administration of the $^{13}C$-$CaCO_3$ suspension (0 minute) and at each point in time (2, 5, 10, 15, 20, 30, 40, 50, and 60 minutes) after the administration of the $^{13}C$-$CaCO_3$ suspension; and $\Delta^{13}C$(%) was measured with a mass spectrometer for expired air analysis (ABCA: manufactured by SerCon) from the concentration of $^{13}CO_2$ excreted in the expired air.

Fig. 6 shows the results. In Fig. 6, -□- and -■- respectively show changes in the $\Delta^{13}C$(‰) in the expired air measured in Group 1 (control group) and Group 2 (model group with decreased gastric acidity) over time.

Fig 6 reveals that the $\Delta^{13}C$ (‰) in the expired air of the rats whose gastric acid secretion had been suppressed by the administration of the PPI (model group with decreased gastric acidity: -■-) was significantly lower than the $\Delta^{13}C$ (‰) in the expired air of the normal rats (control group: -□-).

The above results indicate that even in the case of mammals with decreased gastric acidity due to suppression of gastric acid secretion, there is a linear correlation (linearity) between the dose of the $^{13}C$-labeled carbonate compound in the range of at least not greater than 100 $\mu$mol/kg, and the $^{13}CO_2$ excretion behavior in the expired air ($^{13}CO_2$ concentration ($\Delta^{13}C$(‰)) in the expired air at each point in time for collecting the expired air and "area under the $\Delta^{13}C$(‰)-time curve" (AUC)).

Experimental Example 3

Measurement of gastric acid secretion increase

(1) Preparation of a pentagastrin administration solution

DMSO and 2 ml of physiological saline were added to 7.5 mg of pentagastrin (SIGMA) that has the action of stimulating and increasing gastric acid secretion so that the total amount was 10 ml, to prepare a pentagastrin administration solution at a concentration of 0.75 mg/ml.

(2) Experiment

Fasted rats (male, SD rats) were used as experimental animals.

Two groups, Group 1 (normal rats, n=3) and Group 2 (normal rats, n=3), were provided as control groups. The

$^{13}$C-CaCO$_3$ suspension prepared in Experimental Example 1 was orally administered to Group 1 and Group 2 in an amount of 500 μmol/kg and 1000 μmol/kg, respectively. As model groups with increased gastric acidity, two groups, Group 3 (n=3) and Group 4 (n=3), were provided. The pentagastrin solution (0.75 mg/mL) was intravenously administered (1 mL/kg) to Group 3 and Group 4 to increase gastric acid secretion (production of animal models with increased gastric acid secretion); and the $^{13}$C-CaCO$_3$ suspension was orally administered to Group 3 and Group 4 in an amount of 500 μmol/kg and 1000 μmol/kg, respectively, 1 hour after the administration of the pentagastrin.

In the rats in each group, expired air was collected before the administration of the $^{13}$C-CaCO$_3$ suspension (0 minute) and at each point in time (2, 5, 10, 15, 20, 30, 40, 50, 60, 80, 100, and 120 minutes) after the administration of the $^{13}$C-CaCO$_3$ suspension, and Δ$^{13}$C(‰) was measured with a mass spectrometer for expired air analysis (ABCA: manufactured by SerCon) from the concentration of $^{13}$CO$_2$ excreted in the expired air.

Fig. 7 shows the results. In Fig. 7, -□- and -■- show changes in the $^{13}$CO$_2$ concentration (Δ$^{13}$C(‰)) in the expired air measured in the control groups Group 1 ($^{13}$C-CaCO$_3$ suspension (500 μmol/kg)-administration group) and Group 2 ($^{13}$C-CaCO$_3$ suspension (1000 μmol/kg)-administration group), respectively; -Δ- and -▲- show changes in the $^{13}$CO$_2$ concentration (Δ$^{13}$C(‰)) in the expired air measured in the model groups with increased gastric acidity Group 3 (pentagastrin + $^{13}$C-CaCO$_3$ suspension (500 μmol/kg)-administration group) and Group 4 (pentagastrin + $^{13}$C-CaCO$_3$ suspension (1000 μmol/kg)-administration group), respectively.

Fig. 7 reveals that the $^{13}$CO$_2$ concentration (Δ$^{13}$C(‰)) in the expired air of the rats whose gastric acid secretion had been increased by the administration of the pentagastrin (model groups with increased gastric acidity: -Δ- and -▲-) was significantly high compared to the $^{13}$CO$_2$ concentration (Δ$^{13}$C(‰) in the expired air of the normal rats (control groups: -□- and -■-).

Fig. 8 shows a comparison of correlations between the $^{13}$C-CaCO$_3$ dose (μmol/kg) of 4 to 2500 μmol/kg and the "area under the Δ$^{13}$C(‰)-time curve" for an expired air collection time of 0 to 120 minutes (AUC120) in the control group (-♦-) and the model group with increased gastric acidity (-▲-). Note that the relation observed between the $^{13}$C-CaCO$_3$ dose and the AUC is also found between the $^{13}$C-CaCO$_3$ dose and the Δ$^{13}$C(‰).

As is clear from the results, in the normal model (control group) Group 1 (-♦-), the plots are linear on a straight line passing through the origin in the dose up to 200 μmol/kg, indicating that there is a linear correlation between the dose and the AUC; however, when the dose exceeds 200 μmol/kg, a plateau phenomenon was observed. On the other hand, in the model with increased gastric acidity Group 2 (-▲-), the plots are on a straight line passing through the origin in the dose of at least up to 1000 μmol/kg, indicating that there is a linear correlation between the dose and the AUC.

The above results indicate that even in the case of mammals with increased gastric acidity due to increased gastric acid secretion, there is a linear correlation (linearity) between the dose of the $^{13}$C-labeled carbonate compound in the range of at least not greater than 1000 μmol/kg, and the $^{13}$CO$_2$ excretion behavior in the expired air ( $^{13}$CO$_2$ concentration (Δ$^{13}$C(‰)) in the expired air at each point in time for collecting the expired air and the "area under the Δ$^{13}$C(‰)-time curve" (AUC)). The results also show that in the model with increased gastric acidity, the range of dose (μg/kg) in which a linear correlation (linearity) was observed between the dose and the Δ$^{13}$C(‰), and the range of dose (μg/kg) in which a linear correlation (linearity) was observed between the dose and the AUC were broader than those in the normal model, i.e., a plateau phenomenon was seen in the model with increased gastric acidity at a dose higher than that in the normal model (see Fig. 1).

From the results of Experimental Examples 2 and 3, the possibility was confirmed that gastric acidity can be quantitatively measured by an expiration test that measures $^{13}$CO$_2$ concentration excreted in expired air after administration of $^{13}$C-CaCO$_3$; and that decrease or increase of gastric acid secretion can be evaluated from the dose and $^{13}$CO$_2$ excretion behavior in the expired air ($^{13}$CO$_2$ concentration (Δ$^{13}$C(‰)) in the expired air at each point in time for collecting the expired air and "area under the Δ$^{13}$C(‰)-time curve" (AUC)).

Experimental Example 4

Administration of a $^{12}$C-CaCO$_3$ and $^{13}$C-CaCO$_3$ mixture

(1) Preparation of administration solutions of a $^{12}$C-CaCO$_3$ and $^{13}$C-CaCO$_3$ mixture (10, 20, 50, 100 μmol/4 mL)

A $^{13}$C-CaCO$_3$ suspension (20 mL) at a concentration of 4 μmol/2 mL was prepared by adding, in small portions, a 0.5% CMC aqueous solution (sodium carboxymethyl cellulose dissolved in distilled water for injection) to 10.1 mg of $^{13}$C-CaCO$_3$ (MW:101, manufactured by Cambridge Isotope Laboratory) while kneading the resulting mixture in a mortar.

$^{12}$C-CaCO$_3$ suspensions at concentrations of 6, 16, 46, and 96 μmol/2 mL were prepared by adding, in small portions, the 0.5% CMC solution to calcium carbonate (MW:100, Wako Pure Chemical Industries, Ltd.) while kneading the resulting mixture in a mortar.

The thus-obtained suspensions were mixed in equal amounts to prepare administration solutions of a $^{12}$C-CaCO$_3$ and $^{13}$C-CaCO$_3$ mixture (hereinafter, simply referred to as "mixture administration solutions") at concentrations of 10, 20, 50, and 100 $\mu$mol/4 mL, respectively. A $^{13}$C-CaCO$_3$ solution at a concentration of 4 $\mu$mol/4 mL was prepared by mixing the $^{13}$C-CaCO$_3$ suspension (4 $\mu$mol/2 mL) and the 0.5% CMC solution in equal amounts.

(2) Experiment

Each of the mixture administration solutions at various concentrations prepared above was forcibly administered orally (4 ml/kg) to fasted rats (male, SD rats: n=3) as experimental animals. Expired air was collected before the administration of the mixture administration solutions (0 minute) and at each point in time (2, 5, 10, 15, 20, 30, 40, 60, 80, 100, and 120 minutes) after the administration of the mixture administration solutions, and $\Delta^{13}$C(‰) was determined with a mass spectrometer for expired air analysis (ABCA: manufactured by SerCon) from the concentration of $^{13}$CO$_2$ excreted in the expired air.

Fig. 9 shows the results. Fig. 9 shows changes in the $\Delta^{13}$C(‰) in the expired air after each of the mixture administration solutions was administered to the rats. As is clear from this graph, it is shown that even in the case where $^{12}$C-CaCO$_3$ is added to $^{13}$C-CaCO$_3$ and the resulting mixture is administered, the behavior of the $\Delta^{13}$C(‰) in the expired air is not affected by the $^{12}$C-CaCO$_3$.

Experimental Example 5

Administration of $^{13}$C-CaCO$_3$ and sodium acetate mixtures

(1) Preparation of $^{13}$C-CaCO$_3$ and sodium acetate mixtures (4, 10, 20, 50, and 100 $\mu$mol/4 mL)

A $^{13}$C-CaCO$_3$ suspension (20 mL) at a concentration of 4 $\mu$mol/2 mL was prepared by adding, in small portions, a 0.5% CMC aqueous solution (sodium carboxymethyl cellulose dissolved in distilled water for injection) to 10.1 mg of $^{13}$C-CaCO$_3$ (MW:101, manufactured by Cambridge Isotope Laboratory) while kneading the resulting mixture in a mortar.

Sodium acetate solutions at predetermined concentrations (6, 16, 46, and 96 $\mu$mol/2 mL) were prepared by adding the 0.5% CMC aqueous solution to sodium acetate (MW:82, Wako Pure Chemical Industries, Ltd.).

The thus-obtained solutions were mixed in equal amounts to prepare mixtures of the $^{13}$C-CaCO$_3$ and the sodium acetate (10, 20, 50, and 100 $\mu$mol/4 mL, respectively). A $^{13}$C-CaCO$_3$ solution at a concentration of 4 $\mu$mol/4 mL was prepared by mixing the $^{13}$C-CaCO$_3$ suspension (4 $\mu$mol/2 mL) and the 0.5% CMC solution in equal amounts.

(2) Experiment

Each of the $^{13}$C-CaCO$_3$ and sodium acetate mixtures at various concentrations (4, 10, 20, 50, and 100 $\mu$mol/4 mL) (hereinafter, simply referred to as "mixtures") was forcibly administered orally (4 mL/kg) to fasted rats (male, SD rats: n=3) as experimental animals. Expired air was collected before the administration of the mixtures (0 minute) and at each point in time (2, 5, 10, 15, 20, 30, 40, 60, 80, 100, and 120 minutes) after the administration of the mixtures, and $\Delta^{13}$C(‰) was determined with a mass spectrometer for expired air analysis (ABCA: manufactured by SerCon) from the concentration of $^{13}$CO$_2$ excreted in the expired air.

Fig. 10 shows the results. Fig. 10 shows changes in the $\Delta^{13}$C(‰) in the expired air after each of the mixtures was administered to the rats. As is clear from Fig. 10, it is shown that even in the case where an alkaline material such as sodium acetate, which does not contain $^{13}$C, is added to $^{13}$C-CaCO$_3$ and the resulting mixture is administered, there is no influence on the behavior of the $^{13}$CO$_2$ concentration in the expired air. That is, it is revealed that even in the case where an alkaline material is mixed with $^{13}$C-CaCO$_3$, there is no influence on an expiration test that measures $^{13}$CO$_2$ concentration excreted in expired air after administration of $^{13}$C-CaCO$_3$.

As shown in Experimental Examples 4 and 5, when the $^{12}$C-carbonate compound or sodium acetate was mixed with the $^{13}$C-labeled carbonate compound, there was no influence on the behavior of $^{13}$CO$_2$. This fact indicates that the method of the present invention can be performed using a smaller amount of the $^{13}$C-labeled carbonate compound by adding the $^{12}$C-carbonate compound or sodium acetate. That is, the amount of the $^{13}$C-labeled carbonate compound can be reduced, thereby reducing the cost.

In Experimental Examples 1 to 5 described above, used as mammals were rats, which are experimental animals widely used as mammals with a respiratory system and digestive system (in particular, gastric acid secretion system) that have functions similar to those of a human respiratory system and digestive system. Rats are hitherto used for screening stimulants of gastric acid secretion or gastric acid secretion inhibitors. As shown in Experimental Example 6 below, all of the aforementioned results can be easily extrapolated to humans, and similar results can also be obtained in the case where the above experiments are carried out for humans.

Experimental Example 6

Evaluation of correlations between the dose (mg/body) of $^{13}C$-$CaCO_3$ and the $\Delta^{13}C(‰)$, and between the dose (mg/body) of $^{13}C$-$CaCO_3$ and the "area under the $\Delta^{13}C(‰)$-time curve" (AUC)" (humans)

The same experiment as in Experimental Example 1 was conducted for humans (n=3) with normal gastric acidity.

(1) Preparation of $^{13}C$-$CaCO_3$ administration suspensions

Administration suspensions at various concentrations (400, 300, 200, 100, 50, and 20 mg/50 mL) were prepared by adding, in small portions, a 0.5% CMC aqueous solution (sodium carboxymethyl cellulose dissolved in distilled water for injection) to $^{13}C$-$CaCO_3$ (MW:101, manufactured by Cambridge Isotope Laboratory) while kneading the resulting mixture in a mortar.

(2) Experiment

To human subjects (n=3) who had abstained from food or drink from 21:00 the day before administration, each of the $^{13}C$-$CaCO_3$ suspensions at various concentrations prepared above was orally administered at 8:30 the next morning (50 mL/body). Expired air was collected before the administration of the $^{13}C$-$CaCO_3$ suspensions (before the $^{13}C$-$CaCO_3$ administration) (0 minute) and at each point in time (2, 4, 6, 8, 10, 12, 14, 16, 18, 20, 25, 30, 40, 50, and 60 minutes) after the administration of the $^{13}C$-$CaCO_3$ suspensions (after the $^{13}C$-$CaCO_3$ administration), and $\Delta^{13}C(‰)$ was determined with a mass spectrometer for expired air analysis (ABCA: manufactured by SerCon) from the concentration of $^{13}CO_2$ excreted in the expired air.

Fig. 11 shows changes in the $\Delta^{13}C(‰)$ in the expired air measured after each of the $^{13}C$-$CaCO_3$ suspensions at various concentrations (400, 300, 200, 100, 50, and 20 mg/body) was administered to the human subjects. In Fig. 11, the $\Delta^{13}C(‰)$ in the expired air is plotted on the ordinate, and the expired air collection time (minutes) after the $^{13}C$-$CaCO_3$ administration is plotted on the abscissa. As shown in Fig. 11, it was observed that the $^{13}C$-$CaCO_3$ was metabolized in the body and excreted as $^{13}CO_2$ in the expired air. In addition, a saturation phenomenon was observed when the dose exceeds 200 mg/body (33 $\mu$mol/kg).

Fig. 12 shows relations between the $^{13}C$-$CaCO_3$ suspension dose (mg/body) of 20 mg/body to 200 mg/body and the $\Delta^{13}C(‰)$ in the expired air at each point in time for collecting the expired air ((1) 8 minutes after the administration, (2) 12 minutes after the administration, and (3) 16 minutes after the administration). Fig. 12 reveals that the $\Delta^{13}C(‰)$ in the expired air versus the dose (mg/body) up to 200 mg/body shows a straight line nearly passing through the origin in all expired air samples, regardless of when the expired air was collected. That is, when the expired air collected at least 8 minutes after the administration of the administration preparation in the dose range of at least not greater than 200 mg/body was used as a test sample, a linear correlation was observed between the $^{13}C$-$CaCO_3$ dose (mg/body) and the $\Delta^{13}C(‰)$ in the expired air ($R^2$ values: 0.9 or more).

Fig. 13 shows correlations between the $^{13}C$-$CaCO_3$ suspension dose (mg/body) of 20 mg/body to 200 mg/body and the "area under the $4^{13}C(‰)$-expired air collection time (20 minutes or 60 minutes) curve" (AUC). The graph of the AUC for an expired air collection time of 0 to 20 minutes (AUC20) versus the $^{13}C$-$CaCO_3$ dose (20 to 200 mg/body) is shown on the left side, whereas the graph of the AUC for an expired air collection time of 0 to 60 minutes (AUC60) versus the $^{13}C$-$CaCO_3$ dose (20 to 200 mg/body) is shown on the right side. As is clear from these graphs, the AUCs for an expired air collection time of 0 to 20 minutes and for an expired air collection time of 0 to 60 minutes (AUC20 and AUC60) versus the $^{13}C$-$CaCO_3$ dose (mg/body) up to 200 mg/body show straight lines nearly passing through the origin. That is, linear correlations were observed between the AUCs and the $^{13}C$-$CaCO_3$ dose of at least not greater than 200 mg/body.

The above results indicate that in humans with normal gastric acidity, there is a linear correlation between the dose of the $^{13}C$-labeled carbonate compound in the range of not greater than 200 mg/body and the $^{13}CO_2$ excretion behavior in the expired air ($^{13}CO_2$ concentration ($\Delta^{13}C(‰)$)) in the expired air at each point in time for collecting the expired air and the "area under the $\Delta^{13}C(‰)$ time curve" (AUC)).

As shown in Figs. 12 and 13, there is a linear correlation between the $^{13}C$-$CaCO_3$ suspension dose (mg/body) up to 200 mg/body and the $\Delta^{13}C(‰)$, and there is a linear correlation between the $^{13}C$-$CaCO_3$ suspension dose (mg/body) up to 200 mg/body and the "area under the $\Delta^{13}C(‰)$-time curve" (AUC). Fig. 14 shows a relation between the dose of 20 to 400 mg/body and the $\Delta^{13}C(‰)$ at an expired air collection time of 30 minutes. The results reveal that when the $^{13}C$-$CaCO_3$ dose is not greater than a certain amount (here, not greater than 200 mg/body), there is a linear correlation between the dose and the $\Delta^{13}C(‰)$; however, when the dose exceeds the certain amount, the $\Delta^{13}C(‰)$ value becomes constant (plateau), as shown in Fig. 1 (1).

Experimental Example 7

A correlation between the enzyme activity of CYP2C19 and CYP3A4 and the effect of omeprazole

Although omeprazole, which is a proton pump inhibitor (PPI), is mainly metabolized by hepatic metabolism enzyme CYP2C19, it is also metabolized by CYP3A4 as a bypass pathway. Thus, the effect of omeprazole depends on the metabolic capacity of CYP2C19 and CYP3A4 in a subject. By using the method for measuring gastric acidity of the present invention, a correlation between the enzyme activity of CYP2C19 and CYP3A4 and the effect of omeprazole was evaluated using a CYP2C19 and CYP3A4 inhibitor (ketoconazole).

(1) Preparation of test samples

(i) ketoconazole (CYP2C19 and CYP3A4 inhibitor): A ketoconazole suspension at a concentration of 50 mg/4 mL (23.54 $\mu$mol/4 mL) was prepared by adding, in small portions, a 0.5% CMC aqueous solution to ketoconazole while kneading the resulting mixture in a mortar.

(ii) Omeprazole: Omeprazole was prepared by dissolving omepral for injection in physiological saline so that the concentration was 3 mg/mL.

(iii) $^{13}$C-CaCO$_3$ suspension: A $^{13}$C-CaCO$_3$ suspension at a concentration of 100 $\mu$mol/4 mL was prepared by adding, in small portions, a 0.5% CMC aqueous solution to $^{13}$C-CaCO$_3$ while kneading the resulting mixture in a mortar.

(2) Experiment

Fasted rats (7 weeks old, female, SD rats) were used as experimental animals. The rats were divided into an omeprazole administration group (n=3), a ketoconazole pre-treatment group (n=3), and a control group (n=3).
To the omeprazole administration group, a single dose of the omeprazole (3 mg/kg) was intravenously administered, and the $^{13}$C-CaCO$_3$ suspension (100 $\mu$mol/4 mL) was orally administered at a dose of 4 mL/kg 2 hours after the administration. Expired air was then collected. To the ketoconazole pre-treatment group, a single dose of the ketoconazole (50 mg/kg) was orally administered in advance, a single dose of the omeprazole (3 mg/kg) was intravenously administered 30 minutes after the administration, and the $^{13}$C-CaCO$_3$ suspension (100 $\mu$mol/4mL) was orally administered at a dose of 4 mL/kg 2 hours after the administration of the omeprazole. Expired air was then collected. To the control group (rats that had not been treated), only the $^{13}$C-CaCO$_3$ suspension (100 $\mu$mol/4 mL) was orally administered at a dose of 4 mL/kg, and expired air was collected.
The above expired air was used as test samples, and $\Delta^{13}$C(‰) was measured with a mass spectrometer for expired air analysis (ABCA: manufactured by SerCon) from the concentration of $^{13}$CO$_2$ excreted in the expired air.
Fig. 15 shows the results. In Fig. 15, -x- indicates change in the $^{13}$CO$_2$ concentration ($\Delta^{13}$C(‰)) in the expired air measured in the control group; -■- indicates change in the $^{13}$CO$_2$ concentration ($\Delta^{13}$C(‰)) in the expired air measured in the omeprazole administration group; and -♦- indicates change in the $^{13}$CO$_2$ concentration ($\Delta^{13}$C(‰)) in the expired air measured in the ketoconazole pre-treatment group.
As shown in Fig. 15, in the omeprazole administration group, gastric acid secretion was suppressed, and then $\Delta^{13}$C(‰)) in the expired air after administering the $^{13}$C-CaCO$_3$ was decreased, compared to the control group. Further, by the ketoconazole pre-treatment, $\Delta^{13}$C(‰) in the expired air after administering the $^{13}$C-CaCO$_3$ was decreased. It is believed that this phenomenon indicates that metabolism of the omeprazole in the body was inhibited by the CYP2C19 and CYP3A4 inhibitor (ketoconazole) to enhance the action of suppressing gastric acid secretion, thereby decreasing gastric acidity.
This experiment confirmed that by using the method for measuring gastric acidity of the present invention, the efficacy (effect) of a drug metabolized by CYP2C19 or by CYP2C19 and CYP3A4 in each subject can be evaluated. The effect of omeprazole and that of the below-described clopidogrel vary between individuals. It is suggested that this is partially attributable to involvement of polymorphisms of the drug metabolizing enzyme CYP2C19 gene or/and CYP3A4 gene. Thus, by using the method for measuring gastric acidity of the present invention, the enzyme activity (metabolic capacity) of CYP2C19 alone or both CYP2C19 and CYP3A4 and the presence of genetic polymorphisms of CYP2C19 or/and CYP3A4 in each subject can be evaluated; and the effect of a drug relating to the enzyme activity (metabolic capacity) of CYP2C19 alone or both CYP2C19 and CYP3A4 on a subject (susceptibility of a subject to the drug) can also be evaluated.

Reference Example 1

A correlation between the enzyme activity of CYP2C19 and the effect of clopidogrel

Clopidogrel, an antithrombotic drug, is converted by CYP2C19 and CYP3A4 into an active metabolite, which binds to the P2Y12 receptor expressed on the surface of platelets to inhibit platelet aggregation. Thus, the effect of clopidogrel depends on the metabolic capacity of CYP2C19 and CYP3A4 in a subject. By using the method for measuring gastric acidity of the present invention, a correlation between the activity of CYP2C19 and CYP3A4, and the effect of clopidogrel was evaluated using a CYP2C19 and CYP3A4 inhibitor (ketoconazole).

(1) Preparation of test samples

(i) Ketoconazole (CYP2C inhibitor): A ketoconazole suspension at a concentration of 50 mg/4 mL (23.54 $\mu$mol/4 mL) was prepared by adding, in small portions, a 0.5% CMC aqueous solution to ketoconazole while kneading the resulting mixture in a mortar.
(ii) Clopidogrel: Clopidogrel was prepared by dissolving clopidogrel in a 0.5% CMC aqueous solution so that the concentration was 10 mg/mL.

(2) Experiment

Fasted rats (7 weeks old, female, SD rats) were used as experimental animals. The rats were divided into a clopidogrel administration group (n=3), a ketoconazole pre-treatment group (n=3), and a control group (n=3).
To the clopidogrel administration group, a single dose of the clopidogrel (10 mg/kg) was orally administered, and blood was collected 2 hours after the administration. To the ketoconazole pre-treatment group, a single dose of the ketoconazole (50 mg/kg) was orally administered in advance, a single dose of the clopidogrel (10 mg/kg) was orally administered 30 minutes after the administration, and blood was collected 2 hours after the administration of the clopidogrel.
Using the collected platelet-rich plasma, platelet aggregation by ADP stimulation was measured with a multifunctional platelet aggregation measurement device (manufactured by MC Medical, Inc.).
Fig. 16 shows the results. As is clear from these results, the platelet aggregation inhibitory action of the clopidogrel was reduced by the CYP2C19 and CYP3A4 inhibitor ketoconazole. It is believed that the metabolic activation of the clopidogrel in the body was inhibited by the CYP2C19 and CYP3A4 inhibitor (ketoconazole), resulting in the reduction in the platelet aggregation inhibitory action.
From these results and the results in Experimental Example 6, it is believed that the efficacy (effect) of clopidogrel in each subject can be evaluated by using the method for measuring gastric acidity of the present invention. Further, it is also believed that by using the method for measuring gastric acidity of the present invention, the effect of clopidogrel relating to the enzyme activity (metabolic activity) of CYP2C19 and CYP3A4 on a subject (susceptibility of a subject to clopidogrel) can be evaluated.

**Claims**

1. A method for measuring gastric acidity of a mammal comprising the steps of:

   (1) using, as a test sample, expired air of a mammalian subject excreted at any point in time within 30 minutes after oral administration of a predetermined amount of a $^{13}$C-labeled carbonate compound, measuring behavior of $^{13}CO_2$ in the expired air;
   (2) comparing the behavior of $^{13}CO_2$ (measured $^{13}CO_2$ behavior) obtained in step (1) with the behavior of corresponding $^{13}CO_2$ (reference $^{13}CO_2$ behavior) that has been obtained beforehand in a control mammal; and
   (3) determining the gastric acidity of the mammalian subject based on a difference between the reference $^{13}CO_2$ behavior and the measured $^{13}CO_2$ behavior obtained in step (2),

   wherein the behavior of $^{13}CO_2$ is $\Delta^{13}C$ (‰) as set forth in (c) or AUC as set forth in (d):

   (c) the difference $\Delta^{13}C(‰) = \delta^{13}C_t - \delta^{13}C_0$ between the "ratio of $^{13}CO_2$ amount to $^{12}CO_2$ amount" ($\delta^{13}C_t$) included in the expired air collected at any point in time for collecting the expired air (t) within 30 minutes after the oral administration of the predetermined amount of the $^{13}$C-labeled carbonate compound, and the "ratio of $^{13}CO_2$ amount to $^{12}CO_2$ amount" ($\delta^{13}C_0$) included in the expired air before the oral administration of the $^{13}$C-labeled carbonate compound

(d) AUC "area under the $\Delta^{13}C(‰)$-time curve" calculated by making a graph by plotting the time from the administration of the predetermined amount of the $^{13}$C-labeled carbonate compound to the collection of the expired air on the abscissa and the $\Delta^{13}C(‰)$ on the ordinate.

2. The method according to claim 1, wherein the predetermined amount is 10 mg to 5 g.

3. The method according to any one of Claims 1 to 2, wherein the $^{13}$C-labeled carbonate compound is at least one carbonate compound selected from the group consisting of alkali metal carbonates, alkaline earth metal carbonates, ammonium carbonate, alkali metal hydrogencarbonates, and ammonium hydrogencarbonate.

4. The method according to any one of claims 3 to 4, wherein determination step (3) is a step of determining that the gastric acidity of a mammalian subject is the same as or higher than the gastric acidity of a control mammal when measured $^{13}CO_2$ behavior and reference $^{13}CO_2$ behavior are the same, or determination step (3) is a step of determining that the gastric acidity of a mammalian subject is lower than the gastric acidity of a control mammal when measured $^{13}CO_2$ behavior is lower than reference $^{13}CO_2$ behavior.

5. The method according to any one of claims 1 to 4 for use in (i) evaluation of an effect of a gastric acid reducer, (ii) evaluation of enzyme activity (metabolic capacity) of CYP2C19 or CYP2C19 and CYP3A4, and (iii) evaluation of an effect of a drug metabolized by CYP2C19 or CYP2C19 and CYP3A4, in a mammalian subject.

6. The method according to claim 5, wherein (i) evaluation of an effect of a gastric acid reducer is carried out by performing the following steps (1) to (3) and then performing the following step (4):

(1) using, as a test sample, expired air of a mammalian subject excreted at any point in time within 30 minutes after oral administration of a predetermined amount of a $^{13}$C-labeled carbonate compound, the oral administration being performed after administration of a gastric acid reducer, measuring behavior of $^{13}CO_2$ excreted in the expired air,

(2) comparing the behavior of $^{13}CO_2$ (measured $^{13}CO_2$ behavior) obtained in step (1) with the behavior of corresponding $^{13}CO_2$ (reference $^{13}CO_2$ behavior) measured in a control mammal to which a predetermined amount of a $^{13}$C-labeled carbonate compound has been orally administered beforehand without administering the gastric acid reducer;

(3) determining gastric acidity of the mammalian subject based on a difference between the reference $^{13}CO_2$ behavior and the measured $^{13}CO_2$ behavior obtained above; and

(4) determining the effect of the gastric acid reducer on the mammalian subject using the gastric acidity of the mammalian subject obtained above as an index.

7. The method according to claim 6, wherein
step (3) is a step of determining that the gastric acidity of the mammalian subject is the same as or higher than the gastric acidity of the control mammal when the measured $^{13}CO_2$ behavior and the reference $^{13}CO_2$ behavior are the same, or that the gastric acidity of the mammalian subject is lower than the gastric acidity of the control mammal when the measured $^{13}CO_2$ behavior is lower than the reference $^{13}CO_2$ behavior and
wherein step (4) is a step of determining that the administered gastric acid reducer has no effect on the mammalian subject when the gastric acidity of the mammalian subject measured in step (3) is the same as or higher than the gastric acidity of the control mammal; or that the administered gastric acid reducer has an effect on the mammalian subject when the gastric acidity of the mammalian subject measured in step (3) is lower than the gastric acidity of the control mammal.

8. The method according to any one of claims 6 to 7, wherein the gastric acid reducer is a proton pump inhibitor, an $H_2$ blocker, or an antacid.

9. The method according to claim 5, wherein (ii) evaluation of enzyme activity (metabolic capacity) of CYP2C19 alone or both of CYP2C19 and CYP3A4 or (iii) evaluation of an effect of a drug metabolized by CYP2C19 alone or both of CYP2C19 and CYP3A4 is carried out by performing the following steps (1) to (3) and then performing the following step (4):

(1) using, as a test sample, expired air of a mammalian subject excreted at any point in time within 30 minutes after oral administration of a predetermined amount of a $^{13}$C-labeled carbonate compound, the oral administration being performed after administration of omeprazole or lansoprazole, measuring behavior of $^{13}CO_2$ excreted in the expired air;

(2) comparing the behavior of $^{13}CO_2$ (measured $^{13}CO_2$ behavior) obtained in step (1) with behavior of corresponding $^{13}CO_2$ (reference $^{13}CO_2$ behavior) measured in a control mammal to which a predetermined amount of a $^{13}$C-labeled carbonate compound has been orally administered beforehand without administering omeprazole or lansoprazole;

(3) determining the gastric acidity of the mammalian subject based on a difference between the reference $^{13}CO_2$ behavior and the measured $^{13}CO_2$ behavior obtained above; and

(4) determining the enzyme activity (metabolic capacity) of CYP2C19 alone or both CYP2C19 and CYP3A4 in the mammalian subject or effect of a drug metabolized by CYP2C19 alone or both CYP2C19 and CYP3A4 on the mammalian subject, using the gastric acidity of the mammalian subject obtained above as an index.

10. The method according to claim 9 wherein

step (3) is a step of determining that the gastric acidity of the mammalian subject is the same as or higher than the gastric acidity of the control mammal when the measured $^{13}CO_2$ behavior and the reference $^{13}CO_2$ behavior are the same, or that the gastric acidity of the mammalian subject is lower than the gastric acidity of the control mammal when the measured $^{13}CO_2$ is lower than the reference $^{13}CO_2$, and

comprising as step (4) a step of determining enzyme activity (metabolic capacity) of CYP2C19 alone or both CYP2C19 and CYP3A4 in a mammalian subject, wherein step (4) is a step of determining that the enzyme activity (metabolic capacity) of CYP2C19 alone or both CYP2C19 and CYP3A4 in the mammalian subject is normal or high when step (3) determines that the gastric acidity of the mammalian subject is the same as or higher than the gastric acidity of the control mammal; or that the enzyme activity (metabolic capacity) of CYP2C19 alone or both CYP2C19 and CYP3A4 in the mammalian subject is low when step (3) determines that the gastric acidity of the mammalian subject is lower than the gastric acidity of the control mammal.

11. The method according to claim 10 comprising as step (4) a step of determining effect of a drug metabolized by CYP2C19 alone or both CYP2C19 and CYP3A4 on a mammalian subject, wherein

(a) in the case where the drug metabolized by CYP2C19 alone or both CYP2C19 and CYP3A4 shows an effect before being metabolized, step (4) is a step of determining that the effect of the drug on the mammalian subject is low when step (3) determines that the gastric acidity of the mammalian subject is the same as or higher than the gastric acidity of the control mammal, or that the effect of the drug on the mammalian subject is high when step (3) determines that the gastric acidity of the mammalian subject is lower than the gastric acidity of the control mammal; or

(b) in the case where the drug metabolized by CYP2C19 alone or both CYP2C19 and CYP3A4 shows an effect by metabolization, step (4) is a step of determining that the effect of the drug on the mammalian subject is high when step (3) determines that the gastric acidity of the mammalian subject is the same as or higher than the gastric acidity of the control mammal, or that the effect of the drug on the mammalian subject is low when step (3) determines that the gastric acidit y of the mammalian subject is lower than the gastric acidity of the control mammal.

12. The method according to claim 11, wherein the drug metabolized by CYP2C19 alone or both CYP2C19 and CYP3A4 is any one of those selected from the group consisting of diazepam, omeprazole, lansoprazole, propranolol, and clopidogrel; the drug that exhibits an effect before being metabolized by CYP2C19 alone or both CYP2C19 and CYP3A4 is any one of those selected from the group consisting of diazepam, omeprazole, lansoprazole, and propranolol; and the drug that exhibits an effect by being metabolized by CYP2C19 alone or both CYP2C19 and CYP3A4 is clopidogrel.

**Patentansprüche**

1. Verfahren zur Messung der Magensäure eines Säugerindividuums, das die folgenden Schritte umfasst:

(1) Verwenden von Ausatemluft eines Säugerindividuums als Testprobe, die zu einem beliebigen Zeitpunkt innerhalb von 30 Minuten nach oraler Verabreichung einer festgelegten Menge einer $^{13}$C-markierten Carbonatverbindung ausgeatmet wird, Messen des Verhaltens von $^{13}CO_2$ in der Ausatemluft;

(2) Vergleichen des in Schritt (1) erhaltenen Verhaltens von $^{13}CO_2$ (gemessenes $^{13}CO_2$-Verhalten) mit dem Verhalten von entsprechendem $^{13}CO_2$ (Referenz-$^{13}CO_2$-Verhalten), das zuvor in einem Kontrollsäuger erhalten wurde; und

(3) Bestimmen der Magensäure des Säugerindividuums auf der Basis einer in Schritt (2) erhaltenen Differenz zwischen dem Referenz-$^{13}CO_2$-Verhalten und dem gemessenen $^{13}CO_2$-Verhalten,

wobei das Verhalten von $^{13}CO_2$ $\Delta^{13}C$(‰), wie in (c) erläutert, oder AUC, wie in (d) erläutert, ist:

(c) die Differenz $\Delta^{13}C$(‰) = $\delta^{13}C_t - \delta^{13}C_0$ zwischen dem "Verhältnis der $^{13}CO_2$-Menge zur $^{12}CO_2$-Menge" ($\delta^{13}C_t$), enthalten in der Ausatemluft, die zu einem beliebigen Zeitpunkt zum Sammeln der Ausatemluft (t) innerhalb von 30 Minuten nach der oralen Verabreichung der festgelegten Menge der $^{13}C$-markierten Carbonatverbindung gesammelt wird, und dem "Verhältnis der $^{13}CO_2$-Menge zur $^{12}CO_2$-Menge" ($\delta^{13}C_0$), enthalten in der Ausatemluft vor der oralen Verabreichung der festgelegten Menge der $^{13}C$-markierten Carbonatverbindung

(d) AUC "Fläche unter der $\Delta^{13}C$(‰)-gegen die Zeit-Kurve", berechnet durch Erstellen eines Graphen durch Auftragen der Zeit von der Verabreichung der festgelegten Menge der $^{13}C$-markierten Carbonatverbindung bis zum Sammeln der Ausatemluft auf der Abszisse und des $\Delta^{13}C$(‰) auf der Ordinate.

2. Verfahren nach Anspruch 1, wobei die festgelegte Menge 10 mg bis 5 g beträgt.

3. Verfahren nach einem der Ansprüche 1 bis 2, wobei die $^{13}C$-markierte Carbonatverbindung mindestens eine Carbonatverbindung ist, die aus der aus Alkalimetallcarbonaten, Erdalkalimetallcarbonaten, Ammoniumcarbonat, Alkalimetallhydrogencarbonaten und Ammoniumhydrogencarbonat bestehenden Gruppe ausgewählt ist.

4. Verfahren nach einem der Ansprüche 3 bis 4, wobei der Bestimmungsschritt (3) ein Schritt ist, in dem bestimmt wird, dass die Magensäure eines Säugerindividuums die gleiche wie oder höher als die Magensäure eines Kontrollsäugers ist, wenn das gemessene $^{13}CO_2$-Verhalten und das Referenz-$^{13}CO_2$-Verhalten gleich sind, oder der Bestimmungsschritt (3) ein Schritt ist, in dem bestimmt wird, dass die Magensäure eines Säugerindividuums niedriger als die Magensäure eines Kontrollsäugers ist, wenn das gemessene $^{13}CO_2$-Verhalten niedriger als das Referenz-$^{13}CO_2$-Verhalten ist.

5. Verfahren nach einem der Ansprüche 1 bis 4 für die Verwendung zur (i) Untersuchung einer Wirkung eines Magensäuresenkers, (ii) Untersuchung der Enzymaktivität (Stoffwechselkapazität) von CYP2C19 oder CYP2C19 und CYP3A4 und (iii) Untersuchung einer Wirkung eines durch CYP2C19 oder CYP2C19 und CYP3A4 metabolisierten Arzneimittels in einem Säugerindividuum.

6. Verfahren nach Anspruch 5, wobei (i) die Untersuchung einer Wirkung einer Magensäuresenkers durchgeführt wird, indem die folgenden Schritte (1) bis (3) durchgeführt werden und dann der folgende Schritt (4) durchgeführt wird:

(1) Verwenden von Ausatemluft eines Säugerindividuums als Testprobe, die zu einem beliebigen Zeitpunkt innerhalb von 30 Minuten nach oraler Verabreichung einer festgelegten Menge einer $^{13}C$-markierten Carbonatverbindung ausgeatmet wird, wobei die orale Verabreichung nach der Verabreichung eines Magensäuresenkers durchgeführt wird, Messen des Verhaltens von in der Ausatemluft ausgeatmetem $^{13}CO_2$;

(2) Vergleichen des in Schritt (1) erhaltenen Verhaltens von $^{13}CO_2$ (gemessenes $^{13}CO_2$-Verhalten) mit dem Verhalten von entsprechendem $^{13}CO_2$ (Referenz-$^{13}CO_2$-Verhalten), das in einem Kontrollsäuger gemessen wird, dem eine festgelegte Menge einer $^{13}C$-markierten Carbonatverbindung zuvor ohne Verabreichung des Magensäuresenkers oral verabreicht wurde;

(3) Bestimmen der Magensäure des Säugerindividuums auf der Basis einer vorstehend erhaltenen Differenz zwischen dem Referenz-$^{13}CO_2$-Verhalten und dem gemessenen $^{13}CO_2$-Verhalten und

(4) Bestimmen der Wirkung des Magensäuresenkers auf das Säugerindividuum unter Verwendung der vorstehend erhaltenen Magensäure des Säugerindividuums als Kennzahl.

7. Verfahren nach Anspruch 6, wobei
man in Schritt (3) bestimmt, dass die Magensäure des Säugerindividuums die gleiche wie oder höher als die Magensäure des Kontrollsäugers ist, wenn das gemessene $^{13}CO_2$-Verhalten und das Referenz-$^{13}CO_2$-Verhalten gleich sind, oder dass die Magensäure des Säugerindividuums niedriger ist als die Magensäure des Kontrollsäugers, wenn das gemessene $^{13}CO_2$-Verhalten niedriger als das Referenz-$^{13}CO_2$-Verhalten ist, und
wobei man in Schritt (4) bestimmt, dass der verabreichte Magensäuresenker keine Wirkung auf das Säugerindividuum hat, wenn die in Schritt (3) gemessene Magensäure des Säugerindividuums die gleiche wie oder höher als die Magensäure des Kontrollsäuger ist; oder dass der verabreichte Magensäuresenker eine Wirkung auf das Säugerindividuum hat, wenn die in Schritt (3) gemessene Magensäure des Säugerindividuums niedriger als die Magensäure des Kontrollsäugers ist.

8. Verfahren nach einem der Ansprüche 6 bis 7, wobei es sich bei dem Magensäuresenker um einen Protonenpumpenhemmer, einen $H_2$-Blocker oder ein Antazidum handelt.

9. Verfahren nach Anspruch 5, wobei (ii) die Untersuchung der Enzymaktivität (Stoffwechselkapazität) von CYP2C19 allein oder von sowohl CYP2C19 als auch CYP3A4 und (iii) die Untersuchung einer Wirkung eines durch CYP2C19 allein oder sowohl CYP2C19 als auch CYP3A4 metabolisierten Arzneimittels durchgeführt wird, indem die folgenden Schritte (1) bis (3) durchgeführt werden und dann der folgende Schritt (4) durchgeführt wird:

   (1) Verwenden von Ausatemluft eines Säugerindividuums als Testprobe, die zu einem beliebigen Zeitpunkt innerhalb von 30 Minuten nach oraler Verabreichung einer festgelegten Menge einer $^{13}$C-markierten Carbonatverbindung ausgeatmet wird, wobei die orale Verabreichung nach der Verabreichung von Omeprazol oder Lansoprazol durchgeführt wird, Messen des Verhaltens von in der Ausatemluft ausgeatmetem $^{13}CO_2$;
   (2) Vergleichen des in Schritt (1) erhaltenen Verhaltens von $^{13}CO_2$ (gemessenes $^{13}CO_2$-Verhalten) mit dem Verhalten von entsprechendem $^{13}CO_2$ (Referenz-$^{13}CO_2$-Verhalten), das in einem Kontrollsäuger gemessen wird, dem eine festgelegte Menge einer $^{13}$C-markierten Carbonatverbindung zuvor ohne Verabreichung von Omeprazol oder Lansoprazol oral verabreicht wurde;
   (3) Bestimmen der Magensäure des Säugerindividuums auf der Basis einer vorstehend erhaltenen Differenz zwischen dem Referenz-$^{13}CO_2$-Verhalten und dem gemessenen $^{13}CO_2$-Verhalten und
   (4) Bestimmen der Enzymaktivität (Stoffwechselkapazität) von CYP2C19 allein oder von sowohl CYP2C19 als auch CYP3A4 in dem Säugerindividuum oder der Wirkung eines durch CYP2C19 allein oder sowohl CYP2C19 als auch CYP3A4 metabolisierten Arzneimittels auf das Säugerindividuum unter Verwendung der vorstehend erhaltenen Magensäure des Säugerindividuums als Kennzahl.

10. Verfahren nach Anspruch 9, wobei
    man in Schritt (3) bestimmt, dass die Magensäure des Säugerindividuums die gleiche wie oder höher als die Magensäure des Kontrollsäugers ist, wenn das gemessene $^{13}CO_2$-Verhalten und das Referenz-$^{13}CO_2$-Verhalten gleich sind, oder dass die Magensäure des Säugerindividuums niedriger ist als die Magensäure des Kontrollsäugers, wenn das gemessene $^{13}CO_2$-Verhalten niedriger als das Referenz-$^{13}CO_2$-Verhalten ist, und
    das als Schritt (4) einen Schritt der Bestimmung der Enzymaktivität (Stoffwechselkapazität) von CYP2C19 allein oder von sowohl CYP2C19 als auch CYP3A4 in einem Säugerindividuum umfasst, wobei man in Schritt (4) bestimmt, dass die Enzymaktivität (Stoffwechselkapazität) von CYP2C19 allein oder von sowohl CYP2C19 als auch CYP3A4 in dem Säugerindividuum normal oder hoch ist, wenn Schritt (3) bestimmt, dass die Magensäure des Säugerindividuums die gleiche wie oder höher als die Magensäure des Kontrollsäugers ist; oder dass die Enzymaktivität (Stoffwechselkapazität) von CYP2C19 allein oder von sowohl CYP2C19 als auch CYP3A4 in dem Säugerindividuum niedrig ist, wenn Schritt (3) bestimmt, dass die Magensäure des Säugerindividuums niedriger als die Magensäure des Kontrollsäugers ist.

11. Verfahren nach Anspruch 10, das als Schritt (4) einen Schritt der Bestimmung der Wirkung eines durch CYP2C19 allein oder sowohl CYP2C19 als auch CYP3A4 metabolisierten Arzneimittels auf ein Säugerindividuum umfasst, wobei

    (a) in dem Fall, wenn das durch CYP2C19 allein oder sowohl CYP2C19 als auch CYP3A4 metabolisierte Arzneimittel eine Wirkung zeigt, bevor es metabolisiert wird, man in Schritt (4) bestimmt, dass die Wirkung des Arzneimittels auf das Säugerindividuum niedrig ist, wenn Schritt (3) bestimmt, dass die Magensäure des Säugerindividuums die gleiche wie oder höher als die Magensäure des Kontrollsäugers ist, oder dass die Wirkung des Arzneimittels auf das Säugerindividuum hoch ist, wenn Schritt (3) bestimmt, dass die Magensäure des Säugerindividuums niedriger als die Magensäure des Kontrollsäugers ist; oder
    (b) in dem Fall, wenn das durch CYP2C19 allein oder sowohl CYP2C19 als auch CYP3A4 metabolisierte Arzneimittel eine Wirkung durch Metabolisierung zeigt, man in Schritt (4) bestimmt, dass die Wirkung des Arzneimittels auf das Säugerindividuum hoch ist, wenn Schritt (3) bestimmt, dass die Magensäure des Säugerindividuums die gleiche wie oder höher als die Magensäure des Kontrollsäugers ist, oder dass die Wirkung des Arzneimittels auf das Säugerindividuum niedrig ist, wenn Schritt (3) bestimmt, dass die Magensäure des Säugerindividuums niedriger als die Magensäure des Kontrollsäugers ist.

**12.** Verfahren nach Anspruch 11, wobei das durch CYP2C19 allein oder sowohl CYP2C19 als auch CYP3A4 metabolisierte Arzneimittel ein beliebiges von denjenigen ist, die aus der aus Diazepam, Omeprazol, Lansoprazol, Propanolol und Clopidogrel bestehenden Gruppe ausgewählt sind; das Arzneimittel, das eine Wirkung zeigt, bevor es durch CYP2C19 allein oder sowohl CYP2C19 als auch CYP3A4 metabolisiert wird, ein beliebiges von denjenigen ist, die aus der aus Diazepam, Omeprazol, Lansoprazol und Propanolol bestehenden Gruppe ausgewählt sind; und das Arzneimittel, das eine Wirkung zeigt, indem es durch CYP2C19 allein oder sowohl CYP2C19 als auch CYP3A4 metabolisiert wird, Clopidogrel ist.

**Revendications**

**1.** Méthode de mesure de l'acidité gastrique d'un mammifère, comprenant les étapes suivantes :

(1) utilisation, en tant qu'échantillon d'essai, de l'air expiré d'un sujet mammalien, excrété en n'importe quel point du temps dans les 30 minutes qui suivent l'administration orale d'une quantité prédéterminée d'un carbonate marqué au $^{13}$C, en mesurant le comportement de $^{13}CO_2$ dans l'air expiré ;
(2) comparaison du comportement de $^{13}CO_2$ (comportement de $^{13}CO_2$ mesuré) obtenu dans l'étape (1) avec le comportement du $^{13}CO_2$ correspondant (comportement de $^{13}CO_2$ de référence) ayant été obtenu préalablement chez un mammifère témoin ; et
(3) détermination de l'acidité gastrique du sujet mammalien en se basant sur une différence entre le comportement de $^{13}CO_2$ de référence et le comportement de $^{13}CO_2$ mesuré obtenu dans l'étape (2),

où le comportement de $^{13}CO_2$ est $\Delta^{13}C$(‰) comme décrit en (c) ou AUC comme décrit en (d) :

(c) la différence $\Delta^{13}C$(‰) $= \delta^{13}C_t - \delta^{13}C_0$ entre le « rapport de la quantité de $^{13}CO_2$ sur la quantité de $^{13}CO_2$ » ($\delta^{13}C_t$) inclus dans l'air expiré récupéré en n'importe quel point du temps pour la récupération de l'air expiré (t) dans les 30 minutes qui suivent l'administration orale de la quantité prédéterminée du carbonate marqué au $^{13}$C, et le « rapport de la quantité de $^{13}CO_2$ sur la quantité de $^{12}CO_2$ » ($\delta^{13}C_0$) inclus dans l'air expiré avant l'administration orale du carbonate marqué au $^{13}$C
(d) AUC « aire sous la courbe $\Delta^{13}C$(‰)-temps» calculé en traçant sur un graphique une courbe en portant le temps à partir de l'administration de la quantité prédéterminée du carbonate marqué au $^{13}$C jusqu'à la récupération de l'air expiré en abscisse et $\Delta^{13}C$(‰) en ordonnée.

**2.** Méthode selon la revendication 1, où la quantité prédéterminée est comprise entre 10 mg et 5 g.

**3.** Méthode selon l'une quelconque des Revendications 1 à 2, où le carbonate marqué au $^{13}$C est au moins un carbonate choisi dans le groupe constitué par les suivants : carbonates de métaux alcalins, carbonates de métaux alcalino-terreux, carbonate d'ammonium, hydrogénocarbonates de métaux alcalins, et hydrogénocarbonate d'ammonium.

**4.** Méthode selon l'une quelconque des revendications 3 à 4, où l'étape de détermination (3) est une étape permettant de déterminer si l'acidité gastrique d'un sujet mammalien est identique ou supérieure à l'acidité gastrique d'un mammifère témoin lorsque le comportement mesuré de $^{13}CO_2$ et le comportement de $^{13}CO_2$ de référence sont identiques, ou l'étape de détermination (3) est une étape permettant de déterminer si l'acidité gastrique d'un sujet mammalien est inférieure à l'acidité gastrique d'un mammifère témoin lorsque le comportement mesuré de $^{13}CO_2$ est inférieur au comportement $^{13}CO_2$ de référence.

**5.** Méthode selon l'une quelconque des revendications 1 à 4 pour utilisation dans (i) l'évaluation d'un effet d'un réducteur d'acide gastrique, (ii) l'évaluation de l'activité enzymatique (capacité métabolique) de CYP2C19 ou de CYP2C19 et de CYP3A4, et (iii) l'évaluation d'un effet d'un médicament métabolisé par CYP2C19 ou CYP2C19 et CYP3A4, chez un sujet mammalien.

**6.** Méthode selon la revendication 5, où (i) l'évaluation d'un effet d'un réducteur d'acide gastrique est mise en oeuvre par l'exécution des étapes (1) à (3) suivantes puis l'exécution de l'étape (4) suivants :

(1) utilisation, en tant qu'échantillon d'essai, de l'air expiré d'un sujet mammalien excrété en n'importe quel point du temps dans les 30 minutes qui suivent l'administration orale d'une quantité prédéterminée d'un carbonate marqué au $^{13}$C, l'administration orale étant mise en oeuvre après administration d'un réducteur d'acide gastrique, en mesurant le comportement de $^{13}CO_2$ excrété dans l'air expiré,

(2) comparaison du comportement de $^{13}CO_2$ (comportement mesuré de $^{13}CO_2$) obtenu dans l'étape (1) avec le comportement du $^{13}CO_2$ correspondant (comportement de $^{13}CO_2$ de référence) mesuré chez un mammifère témoin auquel une quantité prédéterminée d'un carbonate marqué au $^{13}C$ a été préalablement administrée par voie orale sans administration du réducteur d'acide gastrique ;

(3) détermination de l'acidité gastrique du sujet mammalien en se basant sur une différence entre le comportement de $^{13}CO_2$ de référence et le comportement de $^{13}CO_2$ mesuré obtenu ci-avant ; et

(4) détermination de l'effet du réducteur d'acide gastrique sur le sujet mammalien en utilisant l'acidité gastrique du sujet mammalien obtenu ci-avant en tant qu'indice.

7. Méthode selon la revendication 6, où l'étape (3) est une étape permettant de déterminer si l'acidité gastrique du sujet mammalien est identique ou supérieure à celle du mammifère témoin lorsque le comportement de $^{13}CO_2$ mesuré et le comportement de référence de $^{13}CO_2$ sont identiques, ou si l'acidité gastrique du sujet mammalien est inférieure à l'acidité gastrique du mammifère témoin lorsque le comportement de $^{13}CO_2$ mesuré est inférieur au comportement de référence de $^{13}CO_2$ et

où l'étape (4) est une étape permettant de déterminer si le réducteur d'acide gastrique administré n'a pas d'effet sur le sujet mammalien lorsque l'acidité gastrique du sujet mammalien mesurée dans l'étape (3) est identique ou supérieure à l'acidité gastrique du mammifère témoin ; ou si le réducteur d'acide gastrique administré a un effet sur le sujet mammalien lorsque l'acidité gastrique du sujet mammalien mesurée dans l'étape (3) est inférieure à l'acidité gastrique du mammifère témoin.

8. Méthode selon l'une quelconque des revendications 6 à 7, où le réducteur d'acide gastrique est un inhibiteur de la pompe à protons, un agent bloquant $H_2$ ou un antiacide.

9. Méthode selon la revendication 5, où (ii) l'évaluation de l'activité enzymatique (capacité métabolique) de CYP2C19 seule ou de CYP2C19 et de CYP3A4 simultanément ou (iii) l'évaluation d'un effet d'un médicament métabolisé par CYP2C19 seule ou CYP2C19 et CYP3A4 simultanément est mise en oeuvre en exécutant les étapes (1) à (3) suivantes puis en exécutant l'étape (4) suivants :

(1) utilisation, en tant qu'échantillon d'essai, de l'air expiré d'un sujet mammalien excrété en n'importe quel point du temps dans les 30 minutes qui suivent l'administration orale d'une quantité prédéterminée d'un carbonate marqué au $^{13}C$, l'administration orale étant mise en oeuvre après administration d'oméprazole ou de lansoprazole en mesurant le comportement de $^{13}CO_2$ excrété dans l'air expiré ;

(2) comparaison du comportement de $^{13}CO_2$ (comportement mesuré de $^{13}CO_2$) obtenu dans l'étape (1) avec le comportement du $^{13}CO_2$ correspondant (comportement de $^{13}CO_2$ de référence) mesuré chez un mammifère témoin auquel une quantité prédéterminée d'un carbonate marqué au $^{13}C$ a été préalablement administrée par voie orale sans administration d'oméprazole ou de lansoprazole ;

(3) détermination de l'acidité gastrique du sujet mammalien en se basant sur une différence entre le comportement de $^{13}CO_2$ de référence et le comportement de $^{13}CO_2$ mesuré obtenu ci-avant ; et

(4) détermination de l'activité enzymatique (capacité métabolique) de CYP2C19 seule ou de CYP2C19 et CYP3A4 simultanément sur le sujet mammalien ou de l'effet d'un médicament métabolisé par CYP2C19 seule ou CYP2C19 et CYP3A4 simultanément sur le sujet mammalien, en utilisant l'acidité gastrique du sujet mammalien obtenue ci-avant en tant qu'indice.

10. Méthode selon la revendication 9, où l'étape (3) est une étape permettant de déterminer si l'acidité gastrique du sujet mammalien est identique ou supérieure à celle du mammifère témoin lorsque le comportement de $^{13}CO_2$ mesuré et le comportement de référence de $^{13}CO_2$ sont identiques, ou si l'acidité gastrique du sujet mammalien est inférieure à l'acidité gastrique du mammifère témoin lorsque le $^{13}CO_2$ mesuré est inférieur à la référence de $^{13}CO_2$ et

comprenant en tant qu'étape (4) une étape de détermination de l'activité enzymatique (capacité métabolique) de CYP2C19 seule ou de CYP2C19 et de CYP3A4 simultanément chez un sujet mammalien, où l'étape (4) est une étape permettant de déterminer si l'activité enzymatique (capacité métabolique) de CYP2C19 seule ou de CYP2C19 et CYP3A4 simultanément chez le sujet mammalien est normale ou supérieure lorsque l'étape (3) détermine que l'acidité gastrique du sujet mammalien est identique ou supérieure à l'acidité gastrique du mammifère témoin ; ou que l'activité enzymatique (capacité métabolique) de CYP2C19 seule ou de CYP2C19 et de CYP3A4 simultanément chez un sujet mammalien, est inférieure lorsque l'étape (3) détermine que l'acidité gastrique du sujet mammalien est inférieure à l'acidité gastrique du mammifère témoin.

11. Méthode selon la revendication 10 comprenant en tant qu'étape (4) une étape de détermination de l'effet d'un médicament métabolisé par CYP2C19 seule ou CYP2C19 et CYP3A4 simultanément chez un sujet mammalien, où

(a) dans le cas où le médicament métabolisé par CYP2C19 seule ou CYP2C19 et CYP3A4 simultanément présente un effet avant d'être métabolisé, l'étape (4) est une étape permettant de déterminer que l'effet du médicament sur le sujet mammalien est faible lorsque l'étape (3) détermine que l'acidité gastrique du sujet mammalien est identique ou supérieure à l'acidité gastrique du mammifère témoin, ou que l'effet du médicament sur le sujet mammalien est élevé lorsque l'étape (3) détermine que l'acidité gastrique du sujet mammalien est inférieure à l'acidité gastrique du mammifère témoin ; ou

(b) dans le cas où le médicament métabolisé par CYP2C19 seule ou CYP2C19 et CYP3A4 simultanément présente un effet par métabolisation, l'étape (4) est une étape permettant de déterminer que l'effet du médicament sur le sujet mammalien est élevé lorsque l'étape (3) détermine que l'acidité gastrique du sujet mammalien est égale ou supérieure à l'acidité gastrique du mammifère témoin, ou que l'effet du médicament sur le sujet mammalien est faible lorsque l'étape (3) détermine que l'acidité gastrique du sujet mammalien est inférieure à l'acidité gastrique du mammifère témoin.

12. Méthode selon la revendication 11, où le médicament métabolisé par CYP2C19 seule ou CYP2C19 et CYP3A4 simultanément est l'un de ceux qui sont choisis dans le groupe constitué par les suivants : diazépam, oméprazole, lansoprazole, propranolol et clopidogrel ; le médicament qui présente un effet avant d'être métabolisé par CYP2C19 seule ou CYP2C19 et CYP3A4 simultanément est l'un de ceux qui sont choisis dans le groupe constitué par les suivants : diazépam, oméprazole, lansoprazole et propranolol ; et le médicament qui présente un effet en étant métabolisé par CYP2C19 seule ou CYP2C19 et CYP3A4 simultanément est le clopidogrel.

[FIG. 1]

(1)

(2)

[FIG. 2]

[FIG. 3]

[FIG. 4]

**AUC120**
Dose up to 200

$y = 100.42x - 34.107$
$R^2 = 0.9992$

**AUC60**
Dose up to 200

$y = 78.256x + 293.74$
$R^2 = 0.9994$

[FIG. 5]

(1)

(2)

[FIG. 6]

[FIG. 7]

[FIG. 8]

[FIG. 9]

[FIG. 10]

[FIG. 11]

[FIG. 12]

[FIG. 13]

[FIG. 14]

[FIG. 15]

[FIG. 16]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 101262891 A
- WO 2007007100 A2
- WO 2008028116 A2
- JP 2009515139 A
- WO 0197863 A1

### Non-patent literature cited in the description

- *Eur. J. Gastroenterol. Hepatol.,* 2009, vol. 21 (3), 266-272
- *Gut,* 1999, vol. 45 (I), I18-I22
- **SASAKI.** 5.1 Application of Stable Isotopes in Clinical Diagnosis. *Journal of Japanese Chemistry,* 107
- *Application of Stable Isotopes in Medicine, Pharmacy, and Biology,* 1975, 149-163
- **NANKODO ; KAJIWARA.** *RADIOISOTOPES,* 1992, vol. 41, 45-48
- **KAJIWARA.** *RADIOISOTOPES,* 1992, vol. 41, 45-48
- **KAJIWARA et al.** *RADIOISOTOPES,* 1992, vol. 41, 331-334